# EUROPEAN PATENT APPLICATION

(11) **EP 4 574 959 A1**
(43) Date of publication of application: **25.06.2025**
(21) Application number: 23854927.3
(22) Date of filing: 17.08.2023
(51) Int. Cl.: C12M 1/00, C12M 1/38, C12N 1/04

(54) **CELL FREEZING SYSTEM, VIAL RACK, AND CELL FREEZING METHOD**

(30) Priority: 19.08.2022 JP 2022131339
(71) Applicant: Healios K.K., Tokyo 100-0006 (JP)
(72) Inventor: INAMORI, Masakazu, Tokyo 100-0006 (JP)
(74) Representative: Hoffmann Eitle
(86) International application number: PCT/JP2023/029703
(87) International publication number: WO 2024/038893

(57) **Abstract**

A vial rack having a block part made of a thermally conductive metal as a main body is applied to a gas-cooling type freezer. The block part of the vial rack has one or more holes for inserting vials. The gas-cooling type freezer cools the block part of the vial rack placed in the cooling chamber in a state in which the vials are inserted thereinto, using cooling gas, and the cooled block part cools the vial and the cell suspension in the vial through an inner face of the hole in the block part. A vial rack of the present invention is suitable to cooling by using gas, since the vial rack has a block part as a main body.

## Description

### TECHNICAL FIELD

The present invention relates to a cell freezing system for suitably freezing cells contained in a vial as a cell suspension, a vial rack for use in the cell freezing system, and a cell freezing method using the cell freezing system.

### BACKGROUND ART

In the development of cellular pharmaceuticals and the like, there is a need to develop a cell freezing system capable of quickly freezing and storing a large amount of cells. In particular, regarding cell cooling, studies have been conducted on how to reduce damage to cells during freezing.

In more detail, first, regarding a temperature drop rate when freezing cells, constant cooling at a specific favorable temperature drop rate (approximately 1°C per minute) is recommended (e.g., Patent Literature 1). The constant cooling reduces intracellular ice crystal formation that occurs in rapid cooling, and also reduces excessive intracellular dehydration that occurs in slow cooling, thereby reducing damage caused by these factors.

However, when a cell suspension is placed in a vial and cooled and frozen in a freezer, the temperature significantly rises due to latent heat released from the cell suspension during freezing (latent heat of solidification). It has been reported that the temperature rise caused by this latent heat hinders constant cooling at a favorable temperature drop rate as described above (e.g., Non-Patent Literature 1).

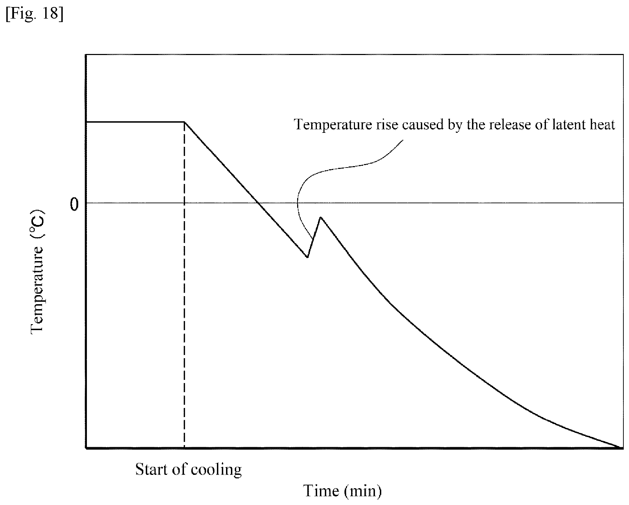

FIG. 18 is a schematic graph diagram of temperature rise caused by the release of latent heat when a cell suspension freezes (also simply referred to as "temperature rise caused by latent heat" hereinafter). When the vial containing the cell suspension is placed in a freezer and constantly cooled at an output that results in a temperature drop rate of about 1°C per minute, latent heat is released from liquid (a solvent of the cell suspension and liquid in the cells) as the liquid freezes, and the temperature of the cell suspension temporarily rises near the freezing point, as shown by a temperature change curve in FIG. 18. Such a temporary temperature rise prevents constant cooling at the above-mentioned favorable temperature drop rate, and cells are damaged due to ice crystals and an increased concentration.

### Citation List

### Patent Literature

Patent Literature 1: US 2005/0241333A1
Patent Literature 2: US Patent No. 7870748B2
Patent Literature 3: US Patent No. 8794013B2

### Non-Patent Literature

Non-Patent Literature 1: Charles J. Hunt, "Technical Considerations in the Freezing, Low-Temperature Storage and Thawing of Stem Cells for Cellular Therapies", Transfus Med Hemother 2019;46:134-149. DOI: 10.1159/000497289

### SUMMARY OF INVENTION

### Technical Problem

In order to subject a large number of vials containing cell suspensions to a freezing process, it is advantageous to use gas-cooling freezer capable of cooling even a large cooling chamber. Further, in order to place a large number of vials in the cooling chamber, it is preferable to use a vial rack. A frame-type vial rack (also referred to as a "frame-type rack" hereinafter) has conventionally been considered preferable as the vial rack (e.g., Patent Literature 2 and the like). In a frame-type rack, like conventionally known test tube racks, the interior of the vial rack is fully open to the outside, thereby allowing body parts of the vials to be fully exposed to the outside (e.g., the racks shown in FIG. 10 of Patent Literature 1 and FIGS. 2 to 5 of Patent Literature 2). That is, it was conceivable that an open structure of the frame-type rack would allow cooling gas to enter the vial rack from the sides, cool the vials, and leave the vial rack, making it possible to provide favorable cooling using gas. A specific configuration example of the frame-type vial rack is the rack shown in FIG. 10 of Patent Literature 1.

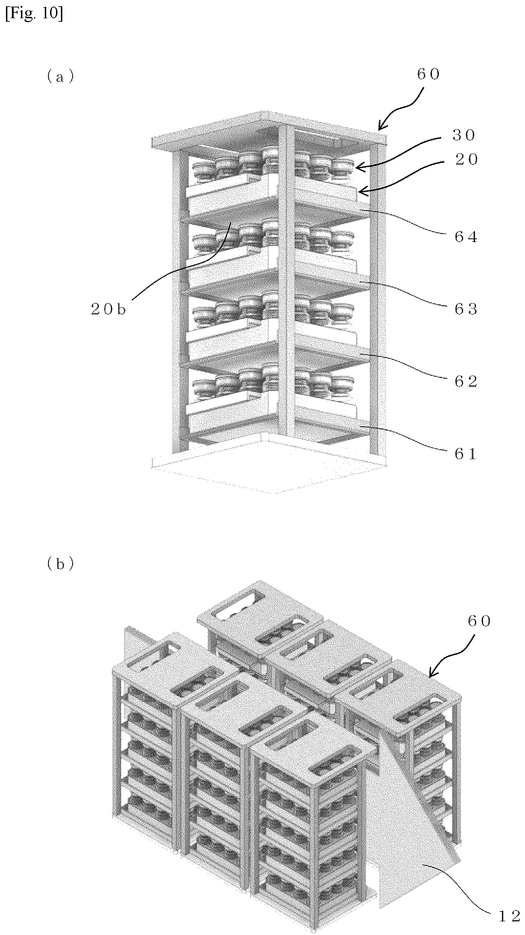

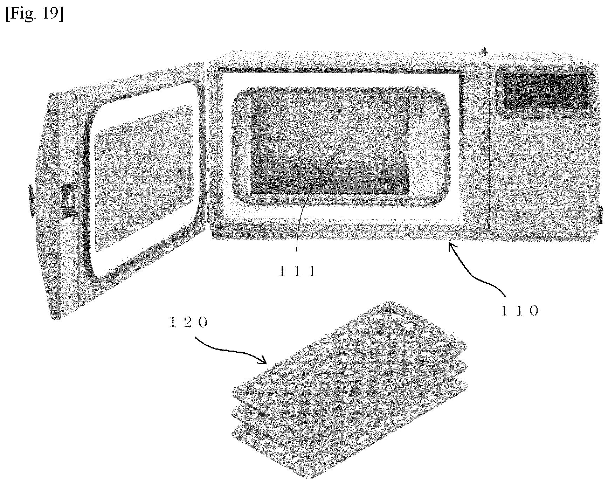

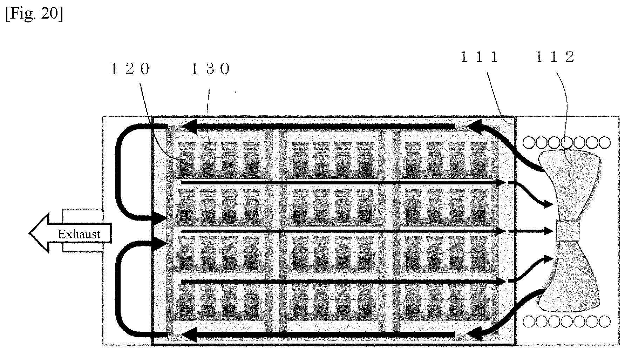

FIG. 19 is a diagram showing an example of a gas-cooling freezer 110 and an example of a frame-type rack 120 to be placed in a cooling chamber 111. The gas-cooling freezer 110 is designed to favorably send low-temperature cooling gas (this low-temperature cooling gas is also referred to as "cold air" hereinafter) capable of freezing a cell suspension into the cooling chamber. On the other hand, the frame-type rack 120 is configured, as the configuration shown in FIG. 10 of Patent Literature 1, such that cold air sent into the cooling chamber preferably flows into the frame-type rack and comes into contact with the vials, and then flows out of the frame-type rack. As shown in FIG. 20 as an example of the flow of cooling gas in the cooling chamber, the gas-cooling freezer 110 operates, using a fan 112, to introduce and circulate cold air whose temperature is lower than the freezing point of the cell suspension into the cooling chamber 111, and blow the cold air onto vials 130 in a frame-type rack 120 to freeze the cell suspension in the vials. Such a freezing process using cold air is specifically described in, for example, Patent Literature 1 and the like.

As described above, conventionally, when a vial rack is to be placed in a gas-cooling freezer, a frame-type rack with good ventilation and having exposed vial body parts needs to be used in consideration of bringing as much of the cold air sent into the cooling chamber into contact with the vials as possible. A combination of such a gas-cooling freezer and a frame-type rack is also described in, for example, Patent Literature 2 mentioned above.

However, the inventors of the present invention have conducted studies in detail on the configurations of cell freezing systems (a configuration using a gas-cooling freezer and a frame-type rack), which were considered to be suitable for mass production as described above, and have found that the following problems arise.

When a temperature rise caused by latent heat is to be suppressed, the timing of the generation of latent heat and the timing of the start of rapid cooling need to be synchronized (e.g., Patent Literature 3). However, when a large number of vials are cooled simultaneously in the same cooling chamber during mass production, the timing of latent heat generation varies from vial to vial. For this reason, it has been found that it is practically difficult to absorb latent heat from a large number of vials all at once through rapid cooling such that the vials have the same temperature. That is, when a large number of vials are cooled simultaneously, the problem of temperature rise caused by latent heat is not fully solved. There is a trade-off relationship between (the increased density of vial arrangement due to efficient mass production) and (the ability to cool a large number of vials evenly or the ability to exchange heat evenly with all the vials). It was found that, if a large number of vials are to be forcibly cooled, then the number of vials whose temperature rise caused by latent heat is not suppressed increases.

As described above, it was found that, in conventional mass production-based cell freezing systems, if a large number of vials are to be cooled at once, cell suspensions in the vials will not have the same temperature, and some vials will contain cell suspensions that have not yet reached the freezing temperature, making it difficult to achieve cooling at a specific temperature drop rate.

It is an object of the present invention to provide a cell freezing system, a vial rack, and a cell freezing method, which have a novel configuration capable of alleviating the above-mentioned temperature-related problems arising when a large number of vials are cooled at once.

### Solution to Problem

Main configurations of the present invention are as follows.
[1] A cell freezing system for freezing a cell suspension by cooling one or more vials containing the cell suspension and inserted into a vial rack,
   the cell freezing system comprising:
   a gas-cooling type freezer having a cooling chamber; and
   the vial rack to be placed in the cooling chamber,
   wherein the vial rack has a block part made of a thermally conductive metal as a main body, the block part having one or more holes for inserting a body part of the vial at least up to a liquid surface of the cell suspension, and
   during use, the gas-cooling type freezer cools the vial rack placed in the cooling chamber in a state in which the vials are inserted into the vial rack, using cooling gas, and the cooled vial rack cools the vial and the cell suspension in the vial through an inner face of the hole in the block part.
[2] The cell freezing system according to [1] above, wherein the thermally conductive metal has a thermal conductivity of 100 (W/(m·K)) or more.
[3] The cell freezing system according to [1] or [2] above, wherein the thermally conductive metal is aluminum, an aluminum alloy, copper, a copper alloy, magnesium, or a magnesium alloy.
[4] The cell freezing system according to any one of [1] to [3] above, wherein a basic shape of the block part of the vial rack is a three-dimensional shape having a flat upper face,
   a plurality of the holes extend from the upper face into the block part, and
   openings of the plurality of holes are positioned on the upper face in a predetermined arrangement pattern.
[5] The cell freezing system according to [4] above, wherein the three-dimensional shape is a rectangular cuboid or a cube.
[6] The cell freezing system according to any one of [1] to [5] above, wherein a basic outer shape of the body part of the vial is round columnar, and a basic shape of an internal space of the hole for inserting the body part is round columnar, and
   a difference between an inner diameter of the hole and an outer diameter of the body part of the vial is 0.1 to 3 mm.
[7] The cell freezing system according to any one of [1] to [6] above, wherein the inner face of the hole is surface treated to have higher emissivity.
[8] The cell freezing system according to any one of [1] to [7] above,
   wherein the hole is a blind hole having a bottom face therein, and
   the inner face of the hole has one or more grooves extending from the opening of the hole to the bottom face, and when the body part of the vial is inserted into the hole, air inside the hole is allowed to escape to an outside through the groove.
[9] The cell freezing system according to [8] above, wherein the groove provided on an inner circumferential surface of the hole and the groove provided in an adjacent hole are integrally connected to each other to form a single slit, thereby allowing adjacent holes to be in communication with each other.
[10] The cell freezing system according to any one of [1] to [9] above, wherein a heat sink is provided on an outer face of the block part to further increase efficiency of heat exchange between the block part and the cooling gas in the cooling chamber.
[11] The cell freezing system according to any one of [1] to [10] above,
   wherein an interior of the block part is provided with one or both of a heat pipe and a vapor chamber to further increase thermal conductivity between an outer face of the block part and a periphery of the hole.
[12] The cell freezing system according to any one of [1] to [11] above, wherein the block part is provided with one or both of a heat pipe and a vapor chamber such that heat of a lower face of the block part is more efficiently conducted to a side face of the block part.
[13] The cell freezing system according to any one of [1] to [12] above, wherein a temperature sensor for measuring a temperature of the block part is attached to the block part of the vial rack, and the temperature sensor is configured to be capable of communicating temperature-related data with a control unit of the gas-cooling type freezer or an external device in a case where the vial rack is placed in the cooling chamber of the gas-cooling type freezer.
[14] The cell freezing system according to any one of [1] to [13] above, wherein the cooling chamber has an interior space sufficiently large to allow a plurality of vial racks to be placed, and
   one or both of a heater and a Peltier element are provided on the block part of each vial rack or at an installation position for each vial rack in the cooling chamber, to allow temperatures of the vial racks to be individually adjustable.
[15] The cell freezing system according to any one of [1] to [14] above,
   wherein the gas-cooling type freezer is a programmable freezer capable of varying cooling capacity according to a preset program, and
   the programmable freezer is configured to increase the cooling capacity during a period in which latent heat is released, to suppress a temperature rise of the cell suspension caused by release of the latent heat when the cell suspension is frozen.
[16] A vial rack for use in the cell freezing system according to any one of [1] to [15] above, the vial rack comprising
   a block part made of a thermally conductive metal as a main body, wherein the block part has one or more holes for inserting the body part of the vial containing the cell suspension at least up to the liquid surface of the cell suspension.
[17] The vial rack according to [16] above, wherein a heat sink is provided on an outer face of the block part to further increase efficiency of heat exchange between the block part and the cooling gas.
[18] The vial rack according to [16] or [17] above, wherein an interior of the block part is provided with one or both of a heat pipe and a vapor chamber to further increase thermal conductivity between an outer face of the block part and a periphery of the hole.
[19] The vial rack according to any one of [16] to [18] above, wherein the block part is provided with one or both of a heat pipe and a vapor chamber to more efficiently conduct heat of a lower face of the block part to a side face of the block part.
[20] A cell freezing method using the cell freezing system according to any one of [1] to [15] above, the method comprising:
   a step of preparing a vial rack for the cell freezing system, a vial containing a cell suspension to be frozen being inserted in the vial rack; and
   a cooling step of cooling the vial rack into which the vial is inserted, in the cooling chamber of the gas-cooling type freezer of the cell freezing system, to cool and freeze the cell suspension,
   wherein, in the cooling step, the gas-cooling type freezer cools the block part of the vial rack to a temperature at which the cell suspension freezes, using cooling gas, and the cooled block part cools the vial and the cell suspension in the vial through the inner face of the hole, thereby freezing the cell suspension.

### Advantageous Effects of Invention

With the cell freezing system and the cell freezing method according to the present invention, instead of blowing cold air in the cooling chamber of the gas-cooling type freezer directly onto vials as in conventional systems, a block part, which is a main body of the vial rack, is cooled by cold air, using a special vial rack made of a thermally conductive metal. The vials are inserted into the holes in the block part, and the inner faces of the holes surround body parts of the vials. Thus, the cooled block part quickly removes heat from the vials and the cell suspension contained therein through thermal conduction and/or thermal radiation. Specifically, in the present invention, the gas-cooling type freezer cools the vial rack using gas, and the vial rack thus cools the vials and their contents through thermal conduction and/or thermal radiation. This preferably suppresses temperature rise caused by latent heat and also preferably suppresses fluctuations in the cooling rate, and thus, even if a large number of vials are placed on one vial rack, the differences in cooling conditions between the vials can be further reduced, and a large number of vials can be cooled more nearly evenly.

**In** the present invention, even if the gas-cooling type freezer is configured to be unable to vary its cooling capacity, if the block part of the vial rack has a sufficiently large heat capacity, latent heat generated from each vial can be favorably absorbed, and the temperatures of the vials can be made uniform. However, in preferred embodiment of the present invention, it is recommended to use a programmable freezer capable of varying its cooling capacity as a gas-cooling type freezer. By using the programmable freezer, the cooling capacity can be increased to match average timing at which the cell suspension in the vials freezes, allowing the latent heat absorbed by the block part of the vial rack from individual vials to be removed more quickly by cold air, and temperature rise caused by latent heat and fluctuations in the cooling rate can be more preferably suppressed.

The vial rack according to the present invention is used to configure the cell freezing system of the present invention, and has a configuration in which holes for inserting vials are provided in the block part made of a thermally (high) conductive metal. With this configuration, simply by cooling the block part made of the thermally conductive metal using cooling gas, the vials (containing freezing targets (objects to be frozen)) inserted into the holes in the block part can be cooled while heat is efficiently removed through the block part made of the thermally conductive metal. As a result, the freezing target can be cooled and frozen while the temperature rise caused by the release of the latent heat is suppressed. The vial rack is favorably used for cooling a large number of vials using low-temperature cooling gas, and its usefulness will become particularly remarkable in use in the cell freezing system and the cell freezing method of the present invention.

### Brief Description of Drawings

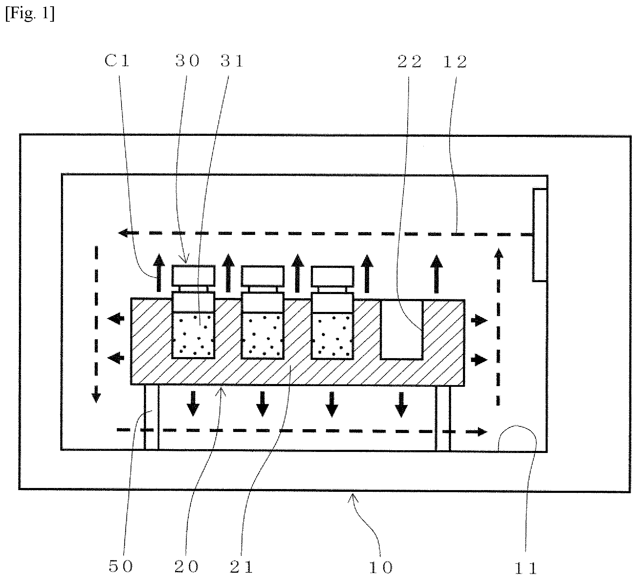

[FIG. 1] FIG. 1 is a cross-sectional view schematically showing an example of the configuration of a cell freezing system and the configuration of a cell freezing method according to the present invention. FIG. 1 shows an external appearance of vials without showing their cross sections (the same applies to the other diagrams). The hatching showing the cross sections is applied only to the vial rack.

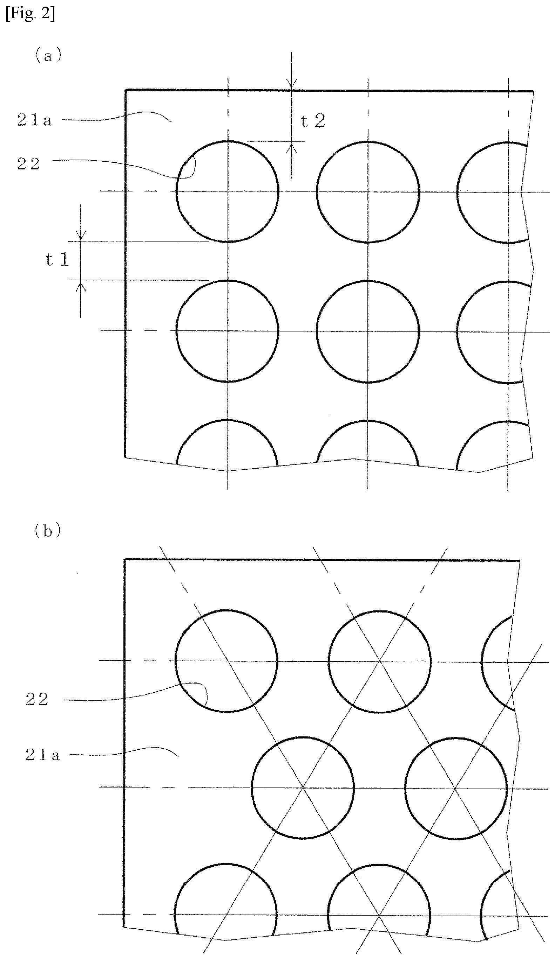

[FIG. 2] FIG. 2 is a diagram showing a portion of an upper face of a block part of the vial rack in the present invention, showing a partial enlarged view illustrating, as an example, an arrangement pattern of holes in the upper face. The dashed-dotted lines are lines passing through the centers of the holes to clearly show the arrangement pattern of the holes.

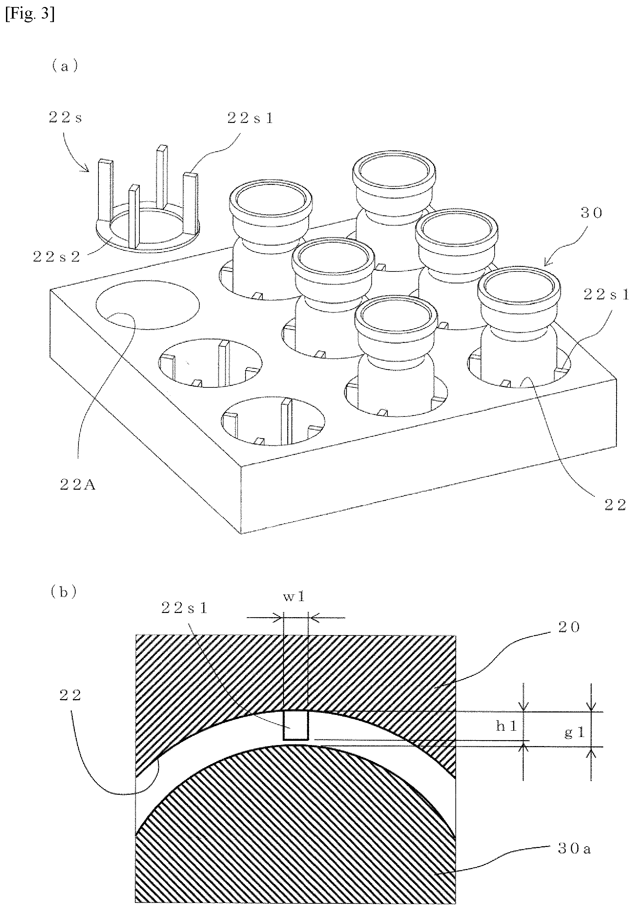

[FIG. 3] FIG. 3(a) is a perspective view and FIG. 3(b) is a partially enlarged view showing a preferred embodiment of the vial rack according to the present invention, showing an example of spacers provided in the holes. In FIG. 3(a), for the purpose of illustration, a spacer is removed from one hole and shown as a sort of assembling/disassembling diagram, and for the purpose of illustration, vials are inserted into six holes. FIG. 3(b) shows a partially enlarged top view of a hole into which the vial is inserted, for the purpose of illustrating dimensions of a protrusion of the spacer. In FIG. 3(b), the block part and the body part of the vial are hatched to more clearly show their regions.

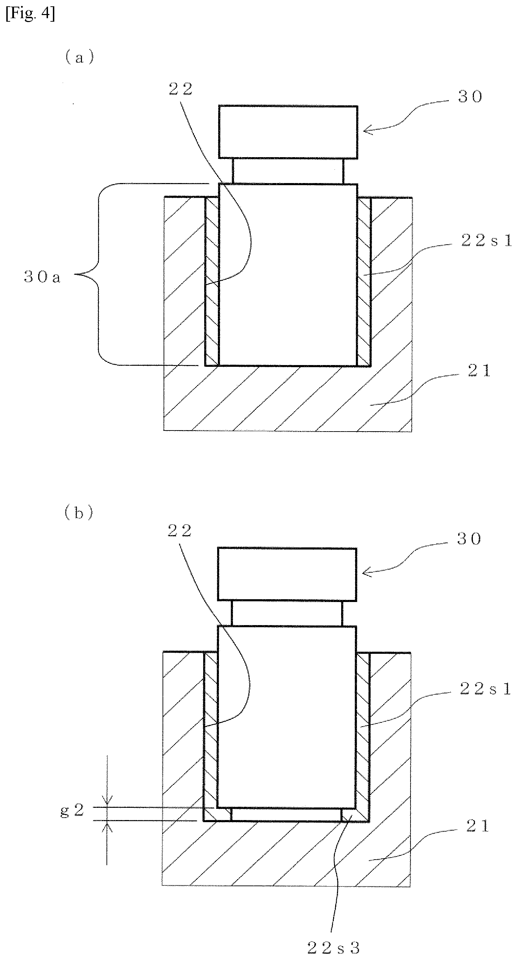

[FIG. 4] FIG. 4 shows a cross-sectional view of a preferred embodiment of the vial rack according to the present invention, showing other examples of a spacer provided in a hole. The hatching showing the cross sections is applied only to the vial rack and protrusions.

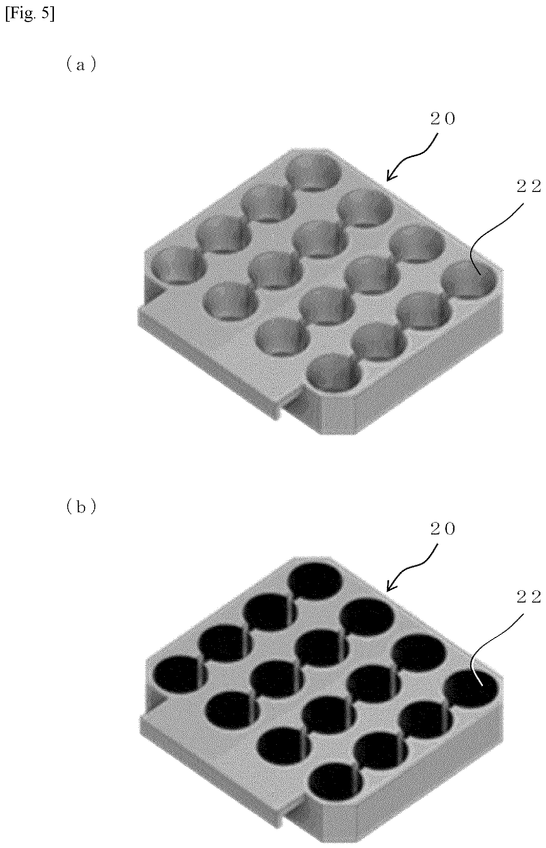

[FIG. 5] FIG. 5 shows a perspective view of a preferred embodiment of the vial rack according to the present invention, showing an example of blackening of inner faces of holes. FIG. 5(a) shows a state where the inner faces of the holes are not blackened, and FIG. 5(b) shows a state where the inner faces of the holes are blackened.

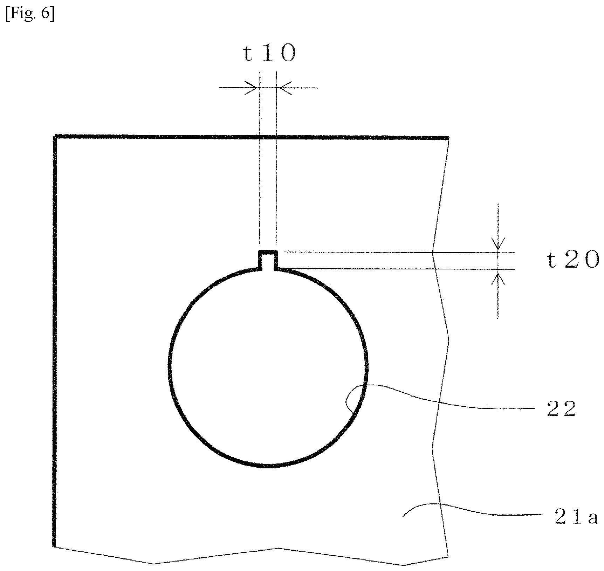

[FIG. 6] FIG. 6 shows a diagram of a portion of the upper face of the block part of the vial rack according to the present invention, and is also a partially enlarged view showing the opening of one hole and its surrounding region, illustrating an example of a groove provided on the inner face of the hole.

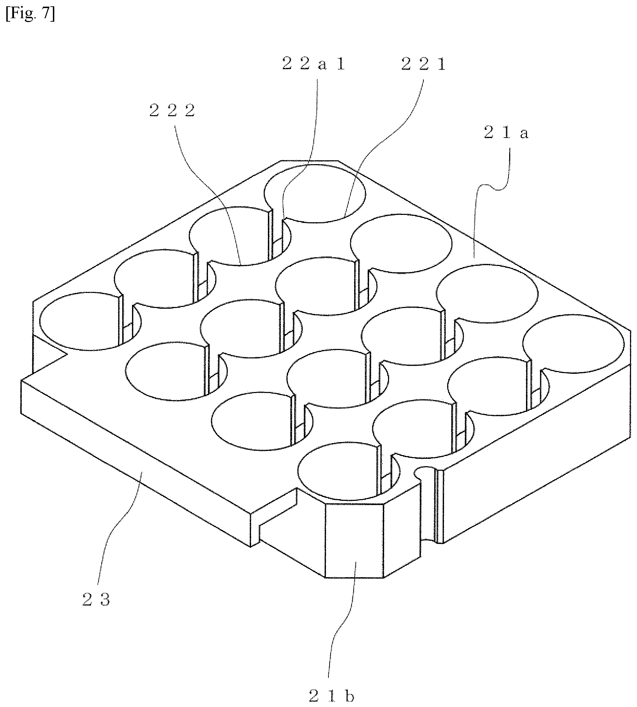

[FIG. 7] FIG. 7 is a perspective view showing an example of a vial rack in which favorable grooves are provided on inner walls of holes according to the present invention.

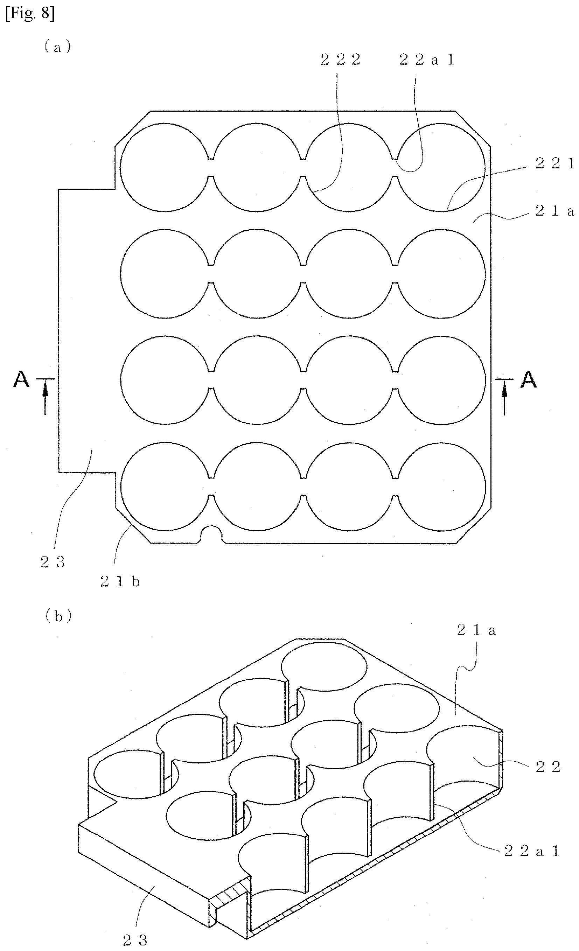

[FIG. 8] FIG. 8 is a diagram illustrating the vial rack shown in FIG. 7 in more detail. FIG. 8(a) shows a plan view of the vial rack, and FIG. 8(b) shows a perspective view of a cross section obtained by cutting the vial rack shown in FIG. 8(a) along a cutting line A-A.

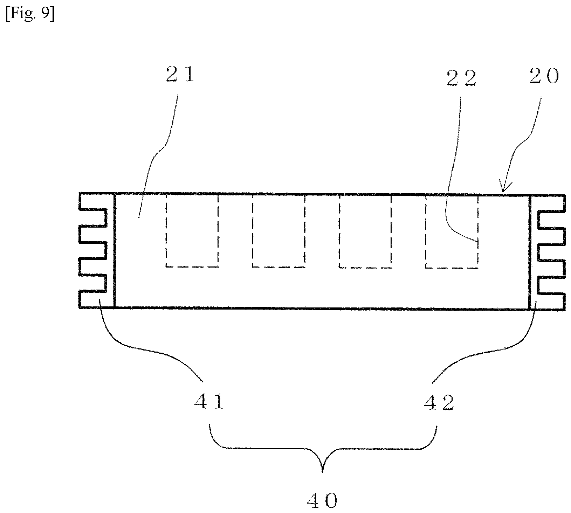

[FIG. 9] FIG. 9 is a schematic diagram showing an example of an embodiment of the present invention in which a heat sink is provided on the outer face of the block part of the vial rack.
[FIG. 10] FIG. 10 is a perspective view showing an example of a multi-tier shelf for placing a large number of vial racks in a cooling chamber according to the present invention. FIG. 10(a) shows a case where there are sufficient gaps between vial racks in the multi-tier shelves. FIG. 10(b) shows a passage of cold air, which is important when the gaps between vial racks in a multi-tier shelf are narrow and a large number of multi-tier shelves are closely packed together.

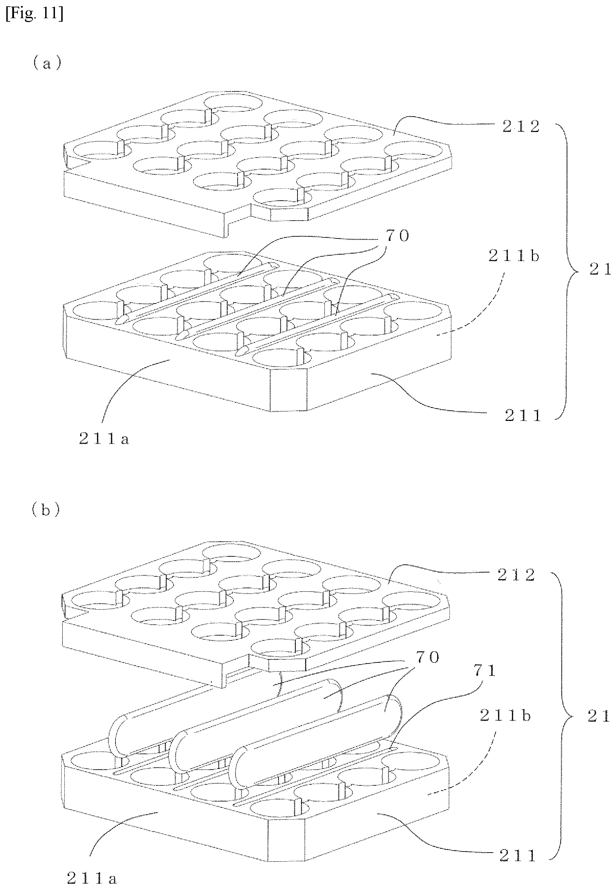

[FIG. 11] FIG. 11 is a schematic diagram showing an example of an embodiment of the present invention in which vapor chambers are provided inside the block part of the vial rack. FIG. 11(a) is an exploded view showing a state in which the vapor chambers are installed in the block part (only a top plate is disassembled and separated to show the inside). FIG. 11(b) is an exploded view showing a state in which the vapor chambers are removed from the block part.

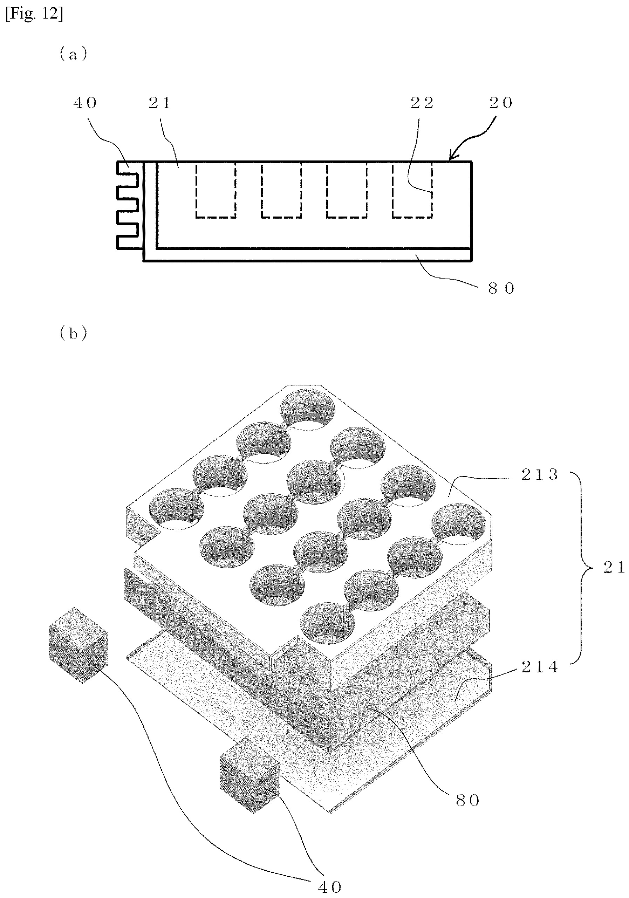

[FIG. 12] FIG. 12 is a diagram illustrating an embodiment of the present invention in which a vapor chamber and a heat sink are provided in the block part of the vial rack. FIG. 12(a) is a side view illustrating an embodiment in which a vapor chamber is provided on a lower face of the block part. FIG. 12(b) is an exploded view showing an embodiment in which the vapor chamber is exposed on the bottoms of the holes in the block part.

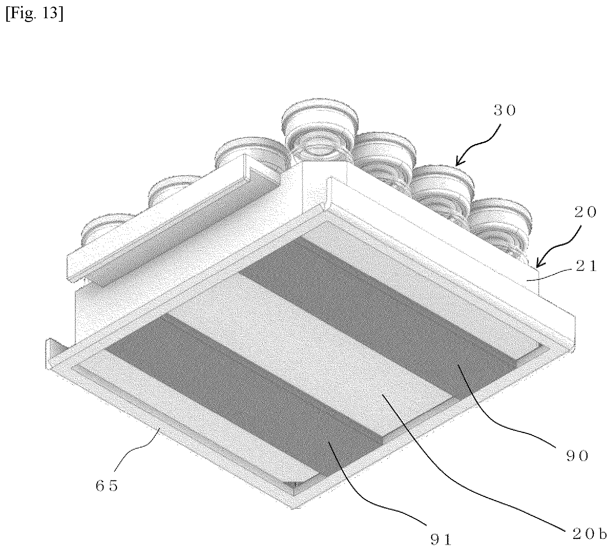

[FIG. 13] FIG. 13 is a perspective view showing an example of an embodiment of the present invention in which heaters and/or Peltier elements are provided such that the temperatures of a plurality of vial racks can be controlled separately.

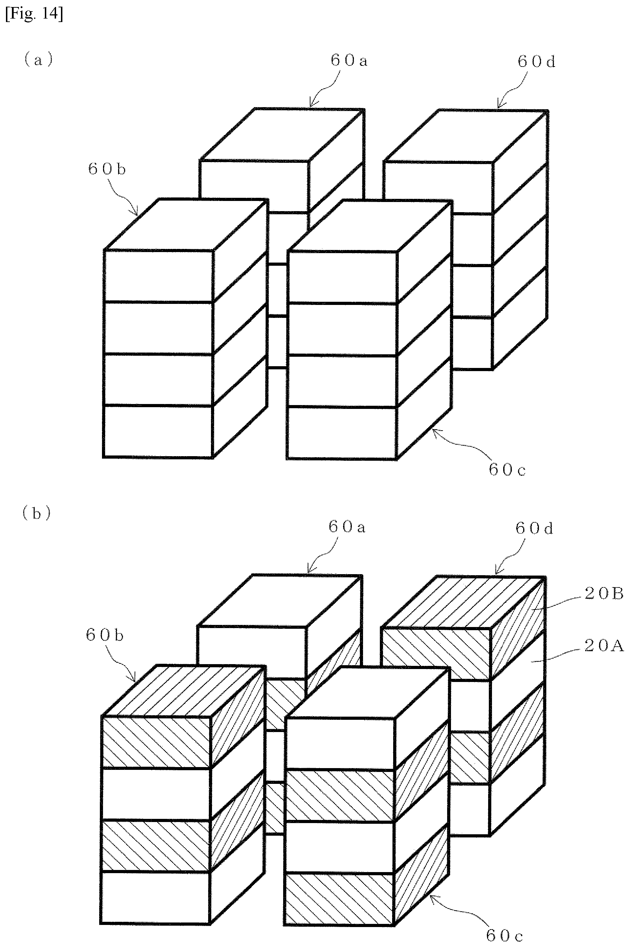

[FIG. 14] FIG. 14 is a schematic diagram showing an arrangement of four-tier shelves in the cooling chamber in Examples of the present invention. In the diagram, each shelf is simply drawn to illustrate the arrangement of the shelves, but the actual shelves are shelves having a frame structure that allows the vial racks placed on each tier to be sufficiently exposed to cold air, as shown in FIG. 10(a), and vial racks are placed on each tier. In FIG. 14(b), for the purpose of distinction, only a vial rack 20B of Comparative Example is hatched.

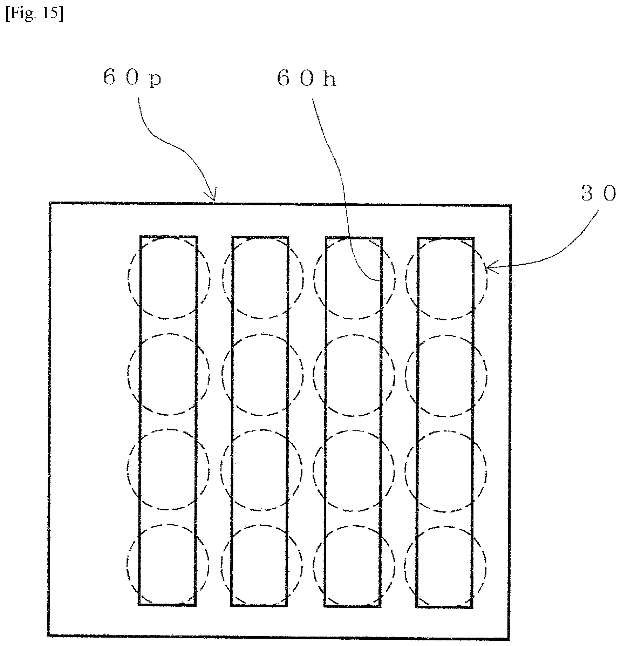

[FIG. 15] FIG. 15 is a schematic diagram showing a configuration of shelf boards of each tier of the shelves placed in the cooling chamber in Examples of the present invention. The diagram shows a board surface with circular body parts of vials drawn overlapping through holes to show a positional relationship between through holes and vials.

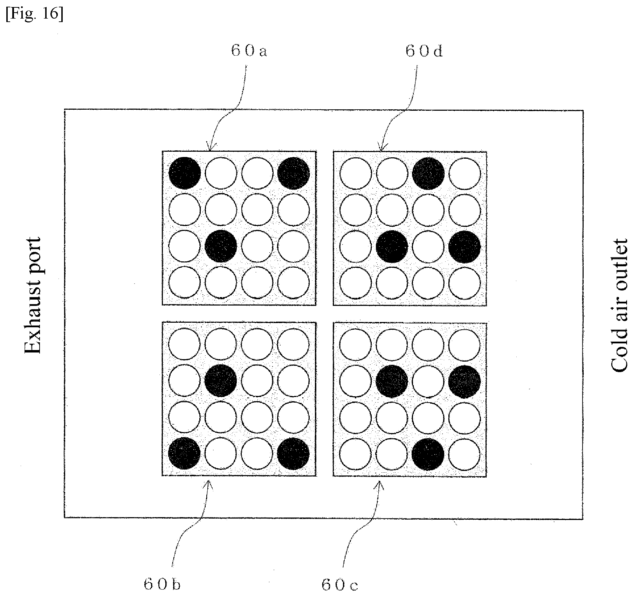

[FIG. 16] FIG. 16 is a schematic diagram showing an arrangement pattern of vials provided with thermocouples for measuring the internal temperature in Examples of the present invention. The diagram shows the upper faces of the vial racks on each of the four shelves when the interior of the cooling chamber is viewed from above.

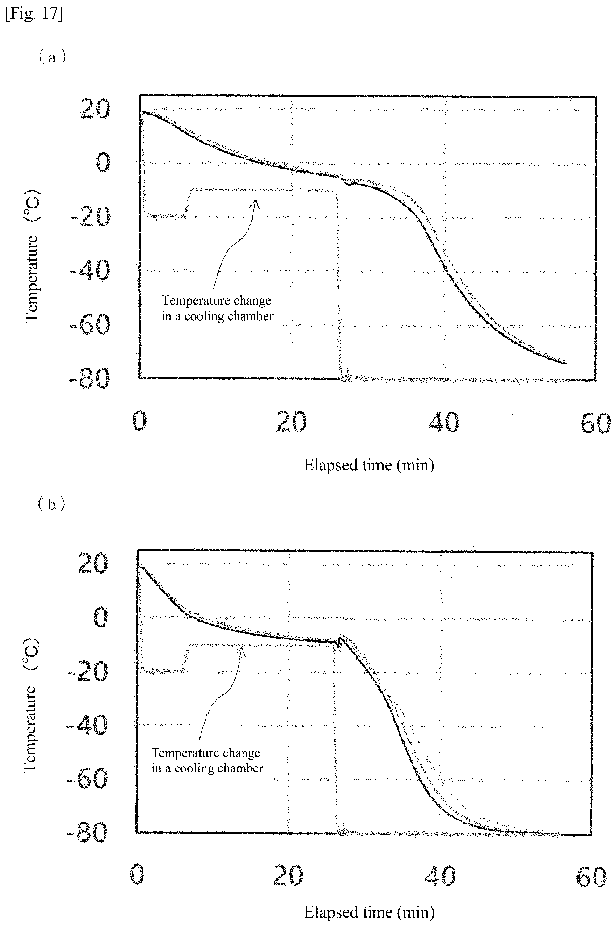

[FIG. 17] FIG. 17 is a graph diagram showing the temperature rise caused by latent heat when vials are cooled using a vial rack according to the present invention and a conventional frame-type vial rack, in Examples of the present invention.
[FIG. 18] FIG. 18 is a graph diagram schematically showing the temperature rise caused by the release of latent heat when a cell suspension freezes.
[FIG. 19] FIG. 19 is a diagram showing an example of a gas-cooling freezer and an example of a frame-type rack placed in a cooling chamber thereof, in a conventional technique.
[FIG. 20] FIG. 20 is a diagram illustrating a flow of cooling gas in a cooling chamber in a combination of a conventional gas-cooling freezer and a frame-type vial rack.

### DESCRIPTION OF EMBODIMENTS

First, a cell freezing system according to the present invention will be described.

As schematically shown in FIG. 1, the cell freezing system is a system for freezing a cell suspension 31 as a result of the gas-cooling type freezer 10 cooling one or more vials 30 containing the cell suspension 31 and inserted into a vial rack 20. Hereafter, an operation such as "cooling a vial containing a cell suspension" will also be simply referred to as "cooling a vial". In such a case, the wording "cooling a vial" also refers to the wording "cooling not only the vials but also the cell suspension therein". The cell freezing system is configured to include the gas-cooling type freezer 10 provided with a cooling chamber 11, and a vial rack 20 placed to be removable from the cooling chamber 11. An openable door for sealing the cooling chamber 11 and a cooling device for supplying cold air (a low-temperature cooling gas capable of freezing the cell suspension) are not shown in the diagram. The vial rack 20 has a block part 21 made of a thermally conductive metal such as aluminum, as a main body. The block part 21 has one or more holes 22 for inserting at least a body part of a vial 30. In FIG. 1, four holes are shown as an example, and a vial is not inserted into one of the four holes in order to clearly show an inner face of the hole. In an actual embodiment, the block part 21 may have a large number of holes arranged in various patterns, like a conventional frame-type rack shown in FIG. 19. The arrangement of the holes may be, for example, compatible with the arrangement of holes in a conventionally known vial rack (in which a large number of holes are arranged at predetermined positions such that vials can be loaded and unloaded by a robotic arm). It is optional whether vials are inserted into all or some of the large number of holes.

When the cell freezing system is in use, the vial rack 20 with the vials 30 inserted therein is placed in the cooling chamber, and a cooling function of the gas-cooling type freezer 10 is activated. As in the example shown in FIG. 1, in the cooling chamber 11 of the gas-cooling type freezer 10, cold air 12 cools the vial rack 20 (particularly the block part 21). Thick arrows C1 indicate heat released from the block part 21 into cold air. Also, a thick dashed line, which indicates the cold air 12, represents the flow of the cold air, but does not limit the path of the flow. The cooled vial rack 20 rapidly cools the vials 30 and the cell suspension 31 therein through an inner face of each hole 22 of the block part 21 (i.e., thermal radiation and/or thermal conductivity). If the temperature of the cold air 12 is low enough to freeze the cell suspension, the cell suspension in each vial will also be rapidly cooled and frozen. As a result, even in a state where a large number of vials are placed, they can be cooled with reduced variation in cooling conditions, and the cell suspension in each vial can be frozen with a preferred temperature drop where the temperature rise caused by latent heat is suppressed and cell damage is reduced.

### Vial

In the present invention, a "vial" is a container that can contain a cell suspension, which is a freezing target (an object to be frozen), and vials include not only a bottle for containing a pharmaceutical product or a cell suspension, but also a container such as a test tube and a centrifuge tube, and also an ample, a syringe, a cryopreservation straw, and a container having no lid. The vial may be designed and produced for the present invention, or may be a commercially available product (including products that meet standards such as ISO 8362-1). There is no particular limitation on the overall shape of a vial and the shape of a body part, and their shapes may be any shape, such as a cuboid or a flat plate. The most ordinary external shape of a body part thereof is round columnar. Hereinafter, the present invention will be specifically described for a case where the external shape of the body part of the vial is a round columnar shape. However, even when the external shape is a shape other than a round columnar shape, the present invention can be implemented by adapting the configuration described below to a configuration in cases other than a round columnar shape.

In the present invention, the "body part of a vial" refers to a main part for containing the cell suspension, and in the example shown in FIG. 4(a), it is a portion below the shoulder of the container, as indicated by reference numeral 30a. Chamfered and round portions at the shoulder and the base may be included in the body part. In shapes having no narrowed opening (an overall straight round columnar shape with no shoulder), the opening may be included in the body part. Also, in the case of a test tube whose body part has a hemispherical lower end, such as a typical round bottom test tube, or a test tube whose body part has a conical lower end, such as a conical test tube, the body part may include the hemispherical or conical lower end.

### Material of Container Part of Vial

The material of a container part of the vial is not particularly limited, and may be a known material that can be used for containing or freezing a cell suspension, such as glass (e.g., borosilicate glass), which is a material of a bottle used to seal medical drugs, or a synthetic resin (e.g., a thermoplastic resin or a thermosetting resin, more specifically, a polyolefin-based resin or a cyclic polyolefin-based resin such as a polypropylene resin, a polyethylene resin, or an ethylenepropylene copolymer, a polystyrene-based resin such as polystyrene or an acrylonitrile-butadienestyrene resin, a methacrylic resin such as a polycarbonate resin, a polyethylene terephthalate resin, or a polymethyl methacrylate resin, a vinyl chloride resin, a polybutylene terephthalate resin, a polyarylate resin, a polysulfone resin, a polyethersulfone resin, a polyether ether ketone resin, a polyetherimide resin, a fluorine-based resin such as polytetrafluoroethylene, a polymethylpentene resin, an acrylic resin such as polyacrylonitrile, and a cellulose-based resin such as propionate resin). Glass is a more preferable material than synthetic resins in that glass has a higher thermal conductivity, but it has a higher risk of breakage during a cooling process or during storage at low temperatures. On the other hand, synthetic resin has lower thermal conductivity than glass, but has a low risk of breakage and is a preferable material for practical use. Among the above synthetic resins, cyclic polyolefin-based resins (hydrogenated ring-opening polymer (COP), cyclic olefin copolymers (COC)) are usually used.

### Vial Lid

As shown in FIG. 1, the vial preferably has a lid capable of sealing its interior, and the lid may have various structures made of various known materials depending on intended use, such as a stopper, a screw cap, a lid that is fixed to the opening of the vial by crimping (e.g., an aluminum crimp top), a plastic cap (e.g., a cap that can be fixed to an opening of the vial by placing the cap over the opening and pressing the cap (RayDyLyo (registered trademark) manufactured by Araymondlife, PLASCAP (registered trademark) manufactured by Daikyo Seiko, Ltd.) and the like), a cap provided with a septum in the center, a cap (AT-Closed Vial (registered trademark) manufactured by Aseptic Technologies) that is punctured with a filling needle, and after the vial is filled with a content, is used for resealing by closing the puncture hole using a laser beam. The opening of the vial may correspond to a sealing structure of the lid, and alternatively, the sealing structure on the inner side of the lid may correspond to the shape of the opening of the vial. A relationship between an outer diameter of the lid and an outer diameter of the body part of the vial varies depending on the capacity of the vial, the structure for sealing the opening of the vial, or the like, and the outer diameter of the lid may be smaller than, the same as, or larger than the outer diameter of the body part of the vial.

### Capacity of Vial and Dimension of Body Part

If the external and internal shapes of the body part of the vial are round columnar, the capacity of the vial (the volume of an internal space of the vial), and external dimensions of the body part (outer diameter and length in the direction of the central axis) are not particularly limited, and a typical capacity is, for example, about 0.2 mL to about 250 mL (mL stands for milliliters). In such a case, the outer diameter of the body part is about 6 mm to about 60 mm, and the length of the body part in the direction of the central axis of the body part is about 20 mm to about 200 mm, for example. Also, a versatile capacity is, for example, about 1 mL to about 50 mL. In such a case, the outer diameter of the body part is about 10 mm to about 50 mm, and the length in the direction of the central axis of the round columnar part is about 35 mm to about 120 mm, for example.

These values are typical examples, and the specifications of the body part of the vial, including the capacity, outer dimensions, wall thickness, and the like, can be freely selected according to the user's requirements or the like. The specifications of the body part of the vial may conform to a vial standard such as ISO 8362-1.

### Cell Suspension as Freezing Target

There is no particular limitation on the cell suspension that can be a freezing target in the present invention, and the present invention will be significantly useful if the cell suspension is a liquid containing dispersed cells that are susceptible to damage caused by temperature rise due to the latent heat generated during freezing. The cell suspension can be produced by, for example, dispersing desired cells in a cell preservation solution, which will be described later.

### Cells

The type of cells that can be used in the present invention is not particularly limited, and may be any of microorganisms, bacteria, animal cells, and plant cells, and animal cells are preferable. Examples of such cells include cells of vertebrates such as mammals (such as mice, rats, guinea pigs, hamsters, rabbits, cats, dogs, sheep, pigs, cows, houses, goats, monkeys, and humans), birds (such as fowls and ostriches), reptiles, amphibians, and fish (such as zebrafish and medaka), and invertebrates such as insects (such as silkworms, moths, and fruit flies). The animal cells are more preferably mammalian cells. When animal cells are used, for example, a cell line of animal cells may be used, cells derived from organs, tissues, or body fluids (including blood, lymph, and semen) collected from animals such as mammals may be used, or transformed cells obtained using a genetic engineering technique may be used. The cells may be either undifferentiated or differentiated cells, and may be either somatic cells or germ cells.

Specific examples thereof include the following cells. Examples of the somatic cells include: (1) tissue stem cells (somatic stem cells) such as neural stem cells, hematopoietic stem cells, mesenchymal stem cells, and dental pulp stem cells; (2) tissue progenitor cells; (3) lymphocytes such as B cells, T cells, and NK cells; and (4) differentiated tissue cells such as epithelial cells, retinal pigment epithelial cells, photoreceptor cells, endothelial cells, muscle cells, fibroblasts (such as skin cells), hair cells, liver cells, gastric mucosal cells, intestinal cells, spleen cells, pancreatic cells (such as exocrine pancreatic cells), nerve cells, brain cells, lung cells, kidney cells, and adipocytes. Examples of the germ cells include sperm, sperm cells, spermatocytes, egg cells, and oocytes. Also, examples of pluripotent stem cells include embryonic stem cells (ES cells), embryonic stem cells derived from cloned embryos obtained through nuclear transfer (ntES cells), germline stem cells (GS cells), embryonic germ cells (EG cells), induced pluripotent stem cells (iPS cells), and pluripotent cells derived from cultured fibroblasts and bone marrow stem cells (Muse cells).

### Cell Preservation Solution

The cell preservation solution that can be used in the present invention is a solution for preserving cells at a low temperature, preferably at -80°C or lower, more preferably -140°C or lower, and even more preferably -196°C or lower. Although not particularly limited, physiological saline, various buffer solutions, culture media for microbial or cell culture, organ preservation solutions, commercially available cryopreservation solutions, and the like can be used alone or in combination. Also, the cell preservation solution may contain serum or serum substitutes, various pharmaceuticals necessary for cell maintenance and the like (e.g., ROCK inhibitors), various vitamins, various amino acids, various cytokines, various hormones, various growth factors, various extracellular matrices, various cell adhesion molecules, antibiotics, stem cell factors, and cryoprotectants, as needed.

Examples of cell culture media include StemFit (e.g., StemFit AK03N, StemFit AK02N) (Ajinomoto Co., Inc.), PECM (Primate ES Cell Medium), GMEM (Glasgow Minimum Essential Medium), IMDM (Iscove's Modified Dulbecco's Medium), 199 medium, Eagle's Minimum Essential Medium (EMEM), αMEM, Dulbecco's modified Eagle's Medium (DMEM), Ham's F12 medium, RPMI 1640 medium, Fischer's medium, and mixtures thereof.

Examples of serum include fetal bovine serum (FBS) and fetal calf serum (FCS). Examples of serum substitutes include substances that contain, as appropriate, albumin (e.g., albumin substitutes such as lipid-rich albumin and recombinant albumin, protein hydrolysates such as plant starch and dextrans), transferrin (or other iron transporters), fatty acids, insulin, collagen precursors, trace elements, 2-mercaptoethanol, 3'-thioglycerol, and equivalents thereof. Such serum substitutes can be prepared using the method described in WO 98/30679, for example. For convenience, commercially available serum substitutes can also be used. Examples of such commercially available substances include Knockout^{™} Serum Replacement (KSR), Chemically-defined Lipid concentrated, and Glutamax (Invitorogen).

Examples of commercially available cryopreservation solutions include STEM-CELLBANKER (registered trademark) (ZENOAQ).

The cryoprotectant that can be used in the present invention refers to a substance that has a high affinity for water molecules and is highly effective in suppressing the growth of ice crystals when dissolved in a cell preservation solution. Specific examples thereof include dimethyl sulfoxide (DMSO), glycerin, trehalose, sucrose, ethylene glycol, polyethylene glycol, propylene glycol, 1,2-propanediol, 1,3-propanediol, butylene glycol, isoprene glycol, dipropylene glycol, polyvinylpyrrolidone, carboxylated polylysine, and poloxamer. The concentration of the cryoprotectant in the cell preservation solution can be set as appropriate depending on the type of cryoprotectant, and may be, for example, 2% by mass to 20% by mass or 5% by mass to 15% by mass.

As an example of a method for preparing a cell suspension, cells such as those listed above as examples are separated from a living organism through enzymatic treatment or the like, or primary cells or cell lines are amplified through culture and, for example, dispersed through enzymatic treatment or the like to obtain the cells, and then a cell preservation solution such as those listed above as examples is added to the obtained cells. During this process, concentration, washing, liquid exchange, or the like may be carried out using a centrifuge, a semipermeable membrane, or the like, and the cell density and the concentration of each component in the suspension may be adjusted as appropriate. The cell suspension may contain one type of cells or a mixture of two or more types of cells. The morphology of the cells in the cell suspension may be in a single cell state, in the form of an aggregate of multiple cells, or in the form of microtissues of a few millimeters or less, such as spheroids. The cell suspension prepared as described above may be dispensed into a sterile container such as those listed above as examples, and may be further closed with a sterile lid such as those listed above as examples. The cells prepared in this manner are subjected to a freezing process performed using the cell freezing system according to the present invention or the like, and are then stored until use.

The cell suspension obtained through such a process is preferably used clinically as a cellular pharmaceutical, and thus it is desirable that the above process is completed under aseptic conditions.

### Vial Rack

As shown in FIG. 1, in the present invention, the vial rack, with the vials inserted therein, is removably positioned within the cooling chamber of the gas-cooling type freezer. The number of vials inserted into the vial rack is one or more, and preferably two or more. A combination of (the dimensions of each part of the vial rack, particularly the dimensions of each part of the block part), (the dimensions, the number, and arrangement pattern of vials to be placed in the block part), and (the dimensions of the cooling chamber and the dimensions of the opening of the cooling chamber) is selected such that the vial rack with vials inserted can enter and exit the space within the cooling chamber.

### Block Part of Vial Rack

The vial rack used in the present invention has the block part (support part) made of a thermally conductive metal, as its main body. The block part has, as an essential element, holes for inserting vials, and in a preferred embodiment, further has additional elements such as a handle (a protrusion for holding) and legs. Therefore, when all the auxiliary elements are removed from the vial rack and the holes are filled with a thermally conductive metal, the basic form of the body (main body) is a metal block (lump, chunk). In the present invention, the metal block, which is the main body, is referred to as a "block part made of a thermally conductive metal". When a vial rack is produced through casting or the like, the block part has holes from the beginning. Because the block part made of a thermally conductive metal has holes for inserting vials, even if the temperature of the cell suspension in the vials rises during cooling, the block part quickly absorbs heat from the vials and the cell suspension through the inner faces of the holes, and the heat is quickly conducted to the outer face of the block part. Heat that has reached the outer face of the block part is absorbed by cold air in the cooling chamber.

### Thermally Conductive Metal and Thermal Conductivity Thereof

The thermally conductive metal that constitutes the block part preferably has a thermal conductivity of 100 (W/(m·K)) or more, more preferably 150 (W/(m·K)) or more, and preferably has as high thermal conductivity as possible. The thermally conductive metal preferably has as high a mechanical strength as possible. According to the "Network Database System for Thermophysical Property Data" operated by the National Institute of Advanced Industrial Science and Technology on its website on the Internet, the thermal conductivity of major thermally conductive metals is approximately as follows: aluminum: 226 to 237, magnesium: 155 to 156, copper: 386 to 402, zinc: 116, gold: 318, silver: 429 (all the values are values near at room temperature, and the unit is (W/(m·K)).

A preferred thermally conductive metal with low cost and good workability is, for example, aluminum, an aluminum alloy, copper, a copper alloy, magnesium, and a magnesium alloy. There is no particular limitation on aluminum alloys, copper alloys, and magnesium alloys as long as they have a thermal conductivity of 100 (W/(m·K)) or more. Representative material symbols (or numbers) of metal materials specified in JIS are listed below as specific examples of preferred metal materials.

### Materials specified in JISH4000:2022 (Aluminum and Aluminum Alloy Sheets, Strips and Plates), JISH4001:2006 (Painted and Baked Aluminum and Aluminum Alloy Sheets and Strips), JISH4040:2015 (Aluminum and Aluminum Alloy Bars and Wires), and JISH4140:1988 (Aluminum and Aluminum Alloy Forgings)

1085 (=A1085), 1080, 1070, 1060, 1050, 1050A, 1N30, 1N99, 1N90, 1100, 1100A, 1200, 1230A, 2011, 2014, 2014A, 2017, 2017A, 2018, 2117, 2218, 2219, 2024, 2025, 2618, 2N01, 2030, 3102, 3003, 3103, 3203, 3021, 3004, 3004A, 3104, 3204, 3005, 4032, 3105, 5005, 5110A, 5021, 5041(5N02), 5042, 5050, 5251, 5052, 5154, 5254, 5454, 5754, 5056, 5456, 5082, 5182, 5083, 5086, 6101, 6151, 6005A, 6005C, 6060, 6061, 6262, 6063, 6181, 6463, 6082, 7003, 7204, 7005, 7020, 7049A, 7010, 7050, 7075, 7N01, 7475, 7178, 8011A, 8021, 8079

In Examples of the present invention, 5052(A5052) is used as the material of the vial rack.

### Materials specified in JISH5202:2010 (Aluminum Alloy Castings) and JISH5302:2006 (Aluminum Alloy Die Castings)

AC1B, AC2A, AC2B, AC3A, AC4A, AC4B, AC4C, AC4CH, AC4D, AC5A, AC7A, AC8A, AC8B, AC8C, AC9A, AC9B, Al Cu4Ti, Al Cu4MgTi, Al Cu5MgAg, Al Si11, Al Si12(a), Al Si12(b), Al Si2MgTi, Al Si7Mg, Al Si7Mg0.3, Al Si7Mg0.6, Al Si9Mg, Al Si10Mg, Al Si10Mg(Cu), Al Si5Cu1Mg, Al Si5Cu3, Al Si5Cu3Mg, Al Si5Cu3Mn, Al Si6Cu4, Al Si7Cu2, Al Si7Cu3Mg, Al Si8Cu3, Al Si9Cu1Mg, Al Si12(Cu), Al Si12CuMgNi, Al Si17Cu4Mg, Al Mg3, Al Mg5, Al Mg5(Si), Al Zn5Mg, Al Zn10Si8Mg, ADC1, ADC3, ADC5, ADC6, ADC10, ADC10Z, ADC12, ADC12Z, ADC14, Al Si9, Al Si12(Fe), Al Si10Mg(Fe), Al Si8Cu3, Al Si9Cu3(Fe), Al Si9Cu3(Fe)(Zn), Al Si11Cu2(Fe), Al Si11Cu3(Fe), Al Si12Cu1(Fe), Al Si17Cu4Mg, Al Mg9

Among the above alloys, AC4B, ADC5, ADC10, ADC10Z, ADC12, ADC12Z, Al Si11Cu3(Fe), and Al Mg9 have different thermal conductivities depending on the material manufacturer, and these alloys can have a thermal conductivity of 100 W/(m·K). The following other alloys are also included in the list as materials that can have a thermal conductivity of 100 W/(m·K) depending on the material manufacturer.

### Materials specified in JISH3100:2018 (Copper and Copper Alloy Sheets, Plates and Strips), JISH3110:2018 (Phosphor Bronze and Nickel Silver Sheets, Plates and Strips), JISH3130:2018 (Copper Beryllium Alloy, Copper Titanium Alloy, Phosphor Bronze, Copper-Nickel-Tin Alloy and Nickel Silver Sheets, Plates and Strips for Springs), JISH3250:2021 (Copper and Copper Alloy Rods and Bars), JISH3260:2018 (Copper and Copper Alloy Wires), and JISH3270:2018 (Copper Beryllium Alloy, Phosphor Bronze and Nickel Silver Rods, Bars and Wires)

C1011, C1020, C1100, C1201, C1220, C1260, C1441, C1510, C1565, C1700, C1720, C1720, C1751, C1862, C1921, C1940, C2100, C2200, C2300, C2400, C2600, C2680, C2700, C2720, C2800, C2801, C3501, C3531, C3601, C3602, C3603, C3604, C3605, C3710, C3712, C3713, C3771, C4250, C4430, C4450, C4621, C4622, C4640, C4641, C5010, C5015, C5050, C5071, C5071, C6782, C6783, C6801, C6802, C6803, C6804, C6810, C6820, C6870, C6871, C6872

### Materials specified in JISH5120:2016 (Copper and Copper Alloy Castings) and JISH5121:2016 (Copper Alloy Continuous Castings)

CAC 101, CAC102, CAC103

### Materials specified in JISH4201:2018 (Magnesium Alloy Sheets, Plates and Strips), JISH4203:2018 (Magnesium Alloy Bars and Wires), and JISH4205:2020 (Magnesium Alloy Forgings)

AZ31B, AZ31C, AZX311, AZ21, ZK30, ZK60, M1, ZM21, ZC71, WZ73, WZ75

### Materials specified in JISH5203:2006 (Magnesium Alloy Castings) and JISH5303:2020

### (Magnesium Alloy Die Castings)

MC6, MC7, MC8, MC9, MC10, MC11, MC14, AM20A

### Materials specified in JISH5301:1990 (Zinc Alloys Die Castings)

ZDC1, ZDC2

Among the above thermally conductive metals, aluminum and aluminum alloys are inexpensive, and block materials that can be used for cutting are widely available, and thus vial racks can be produced at low cost. Also, aluminum and aluminum alloys are highly practical and favorable materials because they are lightweight and corrosion resistant.

When a commercially available metal material is used as a thermally conductive metal, the thermal conductivity provided by the manufacturer of the metal material or the thermal conductivity of the metal material specified in the JIS corresponding to the metal material can be referred to. When unique alloys that require thermal conductivity measurement are to be used, examples of a method for measuring the thermal conductivity of such alloys include the laser flash method, which is a non-steady state method. The laser flash method can be carried out, for example, using a commercially available measurement apparatus (known as a "laser flash method thermal constant measurement apparatus" or the like) and a test piece that is appropriately specified by the manufacturer for each measurement apparatus.

### Basic Shape of Block Part of Vial Rack

Although a basic shape of the block part of the above vial rack is not particularly limited, a three-dimensional shape having a flat surface for arranging hole openings is preferable, and examples thereof include a rectangular cuboid shape, a cubic shape, a round column shape, and a polygonal column shape. The basic shape in the present invention refers to an overall approximate shape presuming that the final shape of the block part has no holes (i.e., presuming that the holes are filled with the material of the block part) and ignoring locally provided recesses and protrusions, chamfered and round corners, and the like. In particular, rectangular cuboids and cubes are preferred shapes because they have a simple shape, are compatible with handling devices such as robots, allow vials to be arranged at a high density, and allow a plurality of vial racks to be arranged without dead space. The flat surface for arranging the hole openings may be not only an upper face, but also a side face or a lower face, and the holes may extend laterally from the side face, or the holes may extend upward from the lower face. In a preferred embodiment in which a vial is held in a hole using gravity and inserted and removed in the up-down direction, similar to conventionally known vial racks, the flat surface is the upper face, and one or more holes, preferably multiple holes, extend downward from the upper face into the block part. Even when the vial rack has the upper face, the orientation of the vial rack during use is not limited, and the vial rack can be used with the upper face facing sideways or downward.

When the basic shape of the block part of the vial rack is a rectangular cuboid or cube, there is no particular limitation on the length of one side and it may be determined as appropriate depending on the number of vials (the number of holes) and their sizes. From the viewpoint of availability of materials, ease of production, the shape and size of a vial to be cooled, the operability of the rack, and the capacity of the programmable freezer, the length of one side is preferably about 5 mm to about 1000 mm, and more preferably about 15 mm to about 500 mm.

### Hole for Inserting Vial

The holes may be through holes, but are preferably blind holes since the block part absorbs latent heat from the lower face of the vial as well. A bottom shape of the blind hole is preferably similar to the shape of the lower face of the body part of the vial. For example, when the basic outer shape of the body part of the vial is round columnar, and the basic shape of an internal space of a blind hole for inserting the body part is also round columnar. A gap between the body part of the vial and the hole will be described later.

### Depth of Hole

Although the depth of a hole may be such that the entire vial enters the hole, preferably, only the body part of the vial enters the hole and its opening protrudes from the hole, from the viewpoint of inserting the vial into the hole and removing the vial from the hole by grasping the lid. From the viewpoint of effectively cooling the cell suspension, it is preferable that the hole has a depth such that the body part of the vial can enter the hole at least up to a liquid surface of the cell suspension. The liquid level will vary depending on the type and the application of the cell suspension and the shape of the vial. For example, in the case of a 10R vial defined in ISO 8362-1, it is preferable that the hole has a depth such that the vial enters the hole by about 85% of the overall height of the container (the dimension from the lower face of the container to the upper face of the opening) from the lower face, which allows the cell suspension in the vial to be effectively cooled and also makes it possible to easily insert and remove the vial by grasping the opening or lid.

### Number of Holes

The number of holes may be one, and in a preferred embodiment for mass production, the number of holes is two or more. The number of holes is not particularly limited, and can be selected as appropriate depending on the outer diameter of the vial, the size of the vial rack, and the like. As vial handling is automated and overall system throughput is increased, the size of the vial rack and the number of holes can be increased accordingly. The number of holes may be, for example, 9 as in the embodiment shown in FIG. 3 (3 columns and 3 rows), 16 as in the embodiment shown in FIG. 7 (4 columns and 4 rows), or may be greater than 78, such as in a conventional vial rack shown in FIG. 19, or even 200 to 300 or more.

### Hole Arrangement Pattern

When a plurality of holes are to be arranged on the flat upper face of the block part, an arrangement pattern of the openings of the holes is not limited. As illustrated in FIG. 2(a) as an example, a matrix-like arrangement pattern is favorable in which center points of the openings of holes 22 on an upper face 21a are located at intersections of a lattice grid (the grid shape is rectangular or square). Further, as illustrated in FIG. 2(b) as an example, a close arrangement pattern in which center points of the openings of the holes are located at the intersections of an equilateral triangular grid (the grid shape is a triangle, in particular, an equilateral triangle), and the like are favorable. The arrangement pattern of the hole openings may be compatible with the arrangement pattern of vial insertion openings in a vial rack used in a conventional cell freezing system. A matrix-like arrangement pattern is preferable because it can simplify a mechanical configuration and control of a robot arm for inserting and removing a vial into/from a hole or the like, and is compatible with the matrix of a table when individual vials are managed in the form of table.

### Thickness of Wall between Adj acent Holes

It is preferable that the thermally conductive metal that surrounds the hole as a wall is sufficiently thick such that heat absorbed by the inner face of the hole from the body part of the vial is more quickly conducted to the outer face of the block part. Thus, when a plurality of holes are arranged, it is preferable that a wall between adjacent holes has a sufficient thickness (a dimension t1 illustrated in FIG. 2(a) as an example). The thickness of the wall between such adjacent holes also varies depending on the inner diameter of the holes, and is preferably 1 mm or more, more preferably 2 mm or more, and particularly preferably about 5 mm to about 10 mm. Although there is no particular limitation on the upper limit of the thickness of the wall, the upper limit is preferably about 10 mm because an excessive thickness is wasteful and the block part of the vial rack is made more compact.

### Thickness of Wall between Side Face of Block Part and Adjacent Hole

Although there is no particular limitation on the thickness of a wall between a side face of the block part and an adjacent hole (a dimension t2 illustrated in FIG. 2(a) as an example), the thickness thereof is preferably about 2 mm to about 5 mm from the standpoint of the mechanical strength of the entire block part and making the block part more compact. Although there is no particular limitation on the thickness of a wall (bottom wall) between the bottom face of the hole and the lower face of the block part (a face opposite to the upper face), the thickness thereof is preferably about 1 mm to about 10 mm, and particularly preferably about 1 mm to about 5 mm, for the following reasons.

The speed of thermal conduction from the bottom face of the hole to the lower face of the block part and the speed of thermal conduction from the side face of the hole to the lower face of the block part through the wall are affected by the surface temperature of the lower face of the block part. Therefore, it is desired that the temperature of the lower face of the block part is uniform. When the thickness of the bottom wall is about 1 mm to about 10 mm, the temperature of the lower face of the block part can be nearly uniform.

Also, when the thickness of the bottom wall is about 1 mm to about 10 mm, the thermal conductivity in a direction parallel to the lower face of the block part in a region between the bottom face of the hole and the lower face of the block part can be uniform, and the mechanical strength of the entire block part can be increased, the block part can be made more compact and lightweight.

### Gap When Vial is Inserted into Hole

When the basic outer shape of the body part of the vial is round columnar, the basic shape of the internal space of a hole for inserting the body part is also round columnar. In this case, it is preferable that a difference between the inner diameter of the hole and the outer diameter of the body part of the vial (this diameter difference is also referred to as a "gap e between the hole and the vial" hereinafter) is as small as possible from the viewpoint of heat transfer. However, in consideration of dimensional errors of the vial, dimensional errors of the hole, and an increase in outer diameter when a label (e.g., with a thickness of about 0.1 mm) is attached to the body of the vial, the gap e between the hole and the vial is preferably about 0.1 mm to about 3 mm, more preferably about 0.1 mm to about 2 mm, even more preferably about 0.1 mm to about 1 mm, and particularly preferably about 0.2 mm to about 0.5 mm. In an example product of the present invention, a length of about 0.2 mm to about 0.4 mm is adopted. By limiting the gap e between the hole and the vial within the above range, the inner face of the hole and the outer face (side face) of the body part of the vial can sufficiently come close to each other and heat transfer therebetween can be mainly thermal conduction and thermal radiation, thereby suppressing uneven cooling and allowing all of the vials to be cooled similarly and quickly. If the gap e between the hole and the vial is more than 3 mm, then the amount of thermal conduction from the body surface to the inner face of the hole may significantly decrease, depending on the outer diameter of the vial.

### Spacer provided in Hole

In a preferred embodiment of the present invention, as shown in FIG. 3(a) as an example, a spacer 22s1 is provided on an inner circumferential surface (side face) of each hole 22 of the vial rack 20 to position the vial 30 coaxially within the hole. In the example shown in FIG. 3(a), spacers 22s1 are provided at four positions spaced apart at equal intervals in the circumferential direction of the hole. Also, in the example shown in FIG. 3(a), the four spacers 22s1 of each hole are integrally connected by a ring-shaped plate 22s2 to form a single spacer member 22s, but each spacer may be independently fixed to the inner circumferential surface of the hole by fitting or bonding. These spacer members 22s are respectively inserted into the holes to provide a spacer to each hole. For the sake of illustration, the diagram shows a state in which the spacer member 22s has been removed from the hole 22A.

### Aspects of Spacer

In the example shown in FIG. 3(a), each spacer 22s1 is a protrusion continuously extending in a depth direction of the hole like a ridge or a square timber, and extends from the opening of the hole to the bottom as shown in FIG. 4(a). Each spacer 22s1 may be not only a ridge-like protrusion, but also a single (i.e., island-like) protrusion that protrudes from the inner circumferential surface of the hole by a predetermined height such that the body part of the vial is positioned coaxially with the hole. By providing the spacer 22s1, inclination of the vial in the hole is suppressed, the distance between the inner face of the hole and the outer face of the vial becomes uniform around the entire circumference of the vial body, thus evenly cooling the vial in each hole.

### Number of Spacers

If the spacer 22s1 is a tubular object that covers the entire inner circumferential surface of the hole 22 with a uniform thickness, such as a bearing bush, it may be difficult to insert or remove the vial and the transfer of heat from the vial to the block part may be hindered, depending on the material of the spacer. Therefore, the spacers 22s1 are preferably protrusions that are spaced apart at about 3 to 6 positions on the inner circumferential surface of the hole at equal intervals in the circumferential direction. If the spacer 22s1 is a protrusion, even if the protruding height of the protrusion is excessively high or even if the outer diameter of the body part of the vial is thick due to manufacturing errors, the protrusion will be crushed by the inserted vial as appropriate, and thus the body part of the vial can be preferably held coaxially with the hole.

A plurality of protrusions that function as spacers may be independently attached to the inner circumferential surface of the hole, or may be connected together by a circular connecting member or the like to form a single member (a single spacer member fitted into the hole), as shown in FIG. 3(a).

### Dimension of Protrusion of Spacer

As shown in FIG. 3(b), a height h1 of a protrusion from the inner circumferential surface of the hole 22 is preferably smaller, by a distance of about 0.05 mm to about 0.2 mm, than a distance g1 between two surfaces (half the gap e between the hole and the vial) that occurs around the entire circumference when the vial is coaxially inserted into the hole. For example, the gap e between the hole and the vial is about 0.1 mm to about 3 mm as described above, the distance g1 between the two surfaces is e/2 = about 0.05 mm to about 1.5 mm, and thus the height h1 of the protrusion in such a case is preferably about 0.01 mm to about 1.3 mm. The width of the protrusion (dimension in the circumferential direction) w1 is not particularly limited, and a preferred dimension is about 1 mm to about 5 mm.

### Material of Spacer

A material of the spacers preferably undergoes little dimensional change due to a temperature change and is unlikely to undergo brittle fracture at low temperatures. Examples thereof include the above-mentioned thermally conductive metals that can be used as materials for vial racks, metal materials for structures, such as steel and stainless steel, and engineering plastics (polyacetal, polyamide, polycarbonate, polyphenylene sulfide, and the like).

### Relationship between Outer Lower Face of Vial and Bottom Face of Hole

It is preferable that the lower face of the vial and the bottom face of the hole are in contact with each other, because a larger amount of heat within the vial can be conducted to the block part. On the other hand, since there is the gap e between the side face of the vial and the hole, there may also be a gap between the lower face of the vial and the bottom face of the hole, similar to the gap e on the side face. Thus, in a preferred embodiment of the present invention, as shown in FIG. 4(b) as an example, a spacer 22s3 is provided to create a gap g2 between the lower face of the body part of the vial and the bottom face of the hole. The gap g2 may be similar to the distance g1 between two surfaces that occurs around the entire circumference when the vial is coaxially inserted into the hole (half of the gap e between the hole and the vial), and may be, for example, about 0.05 mm to about 1.5 mm.

In the example shown in FIG. 4(b), the spacer 22s3 extends laterally from a lower end of the spacer 22s1 on the side face toward the center of the bottom face of the hole by an appropriate length. The spacer 22s3 may be connected in an annular shape, similar to the ring-shaped plate 22s2 of the spacer member 22s shown in FIG. 3(a) as an example. Also, the spacer 22s3 may be a protruding body or a mesh-like body provided on the bottom face of the hole. If the spacer 22s3 covers the entire bottom face of the hole, then the transfer of heat from the bottom of the vial to the block part may be hindered depending on the material. Also, if the entire bottom face is covered by the thermally conductive metal, the hole would simply become shallower. Therefore, in order to provide a gap between the lower face of the vial and the bottom face of the hole, it is preferable that the bottom face of the hole is sufficiently exposed.

### Surface Treatment on Inner Face of Hole for Increasing Emissivity

Regarding the amount of heat transferred, usually (thermal conduction > thermal radiation) holds true, and in particular, in the transfer of heat between an object to be cooled and cold air under convection of the cold air, thermal conduction is dominant and thermal radiation can be ignored. However, in a narrow and windless environment such as the gap e between the hole and the vial, thermal conduction is small and the influence of thermal radiation cannot be ignored.

Thermal radiation is proportional to emissivity ε (between 0 and 1) of a material surface, and an ideal black-body has an emissivity ε of 1. The closer the emissivity ε of the inner face of the hole is to 1, the more heat emitted from the vial is absorbed by the inner face of the hole. In general, the emissivity ε of a metal surface is smaller than 0.1. For example, if the metal surface is colored, for example, black, the emissivity ε of the colored surface will be larger than 0.9, and the amount of heat absorbed by the surface will be increased nearly tenfold. The emissivity ε of the surface may be further increased by further roughening the surface.

**In** view of this, in a preferred embodiment of the present invention, the inner face (the inner circumferential surface and the bottom face) of the hole are subjected to surface treatment for further increasing the emissivity. This is preferable because a larger amount of heat will be transferred from the vial to the block part through the inner face of the hole.

Examples of the surface treatment for the inner face of the hole include anodizing, coloring such as dyeing, black plating, painting, oxide film formation, deposition film formation, and roughening. Roughening can be used in combination with other treatments. FIG. 5 shows an example in which the entire inner face of the hole is colored, where FIG. 5(a) shows a state before coloring and FIG. 5(b) shows a state after coloring.

According to the spectral radiance of a black-body based on Planck's law, it is known that infrared rays with a wavelength of 3 µm or more are mainly emitted from the black-body in a range from room temperature to -80°C. **In** order to improve a cooling effect through radiation, it is important to increase the emissivity, particularly for infrared rays near a wavelength of 3 to 30 µm. Typical surface treatments that can be expected to provide favorable radiation in this wavelength range include the following.

Anodizing: Anodizing coating process for the surface of aluminum or an aluminum alloy. Oxide film (Al₂O₃) exhibits favorable infrared emissivity. Because the oxide film is porous, it is expected that the infrared emissivity can be improved by coloring the oxide film with a colored pigment, dye, or the like, as appropriate. Note that the thermal conductivity of the oxide film (Al₂O₃) is lower than that of aluminum, it is preferable to select an optimal thickness of the oxide film to maximize the effect of thermal conduction and thermal radiation, but it is expected that a thin oxide film also has a sufficient effect.

Heat-dissipating paint: Paints (containing metal oxides, silica, resins, or the like) for facilitating thermal radiation are commercially available, and can improve the emissivity of metals having low emissivity by applying them to the metal surfaces.

Although there is no particular limitation on the thickness of a coating film formed through surface treatment, when the coating film is significantly thick, then the original hole diameter need only be increased in consideration of the thickness of the coating film.

### Hole Air Vent Groove

When the hole is a blind hole having an internal bottom face, it is preferable to provide one or more grooves 22a on the inner face (side face) of the hole 22 from the opening of the hole to the bottom face, as shown in an example of FIG. 6. Even when the gap e between the hole and the vial is small, by providing the grooves 22a, the air in the hole 22 is released to the outside through the grooves 22a when the vial is inserted into the hole 22, and thus the vial can be more smoothly inserted into the hole. It is preferable that the width of the groove (a dimension t10 in FIG. 6) and the depth (a dimension t20 in FIG. 6) of the groove from the inner face of the hole vary depending on the inner diameter of the hole and are not limited, and are each preferably about 1 mm to about 5 mm.

### Air Vent Grooves Connect Holes

FIGS. 7 and 8 show a preferred example of the vial rack according to the present invention. In the example shown in these diagrams, a groove provided on the inner circumferential surface of one hole (hole indicated by reference numeral 221) and a groove provided in an adjacent hole (hole indicated by reference numeral 222) are integrally connected to each other to form a single slit 22a1, and the adjacent holes (221, 222) are thus in communication with each other by the slit. With this configuration, the space within each hole is in communication with the external space on the side of the block part of the vial rack through the slit. Therefore, this configuration is preferable because when the inside of the hole is to be cleaned, cleaning liquid injected into the hole readily flows out through the slits even when the holes have a deep overall shape, and thus cleaning is facilitated.

### Additional Structure of Vial Rack

In a preferred embodiment, as shown in FIGS. 7 and 8, the block part of the vial rack is provided with a handle 23 to aid in handling the vial rack. Corners of the block part of the vial rack may be chamfered greatly as indicated by reference numeral 21b to facilitate insertion into a dispenser or the like.

### Method for Producing Vial Rack

A method for producing the vial rack may be not only a subtractive production method in which the block part made of a thermally conductive metal is first prepared and holes are then formed therein, but also an additive production method in which the block part is formed by casting using a die, forging, or depositing a member made of a thermally conductive metal around portions, which will be holes, and it is possible to use any production method by which the block part having holes can be formed. Also, in order to produce the vial rack, it is possible to use a combination of various processing methods, such as forming a rough shape by casting and improving the dimensional accuracy by cutting.

### Configuration for Improving Heat Exchange on Outer Face of Block Part

In a preferred embodiment of the present invention, as an example of the configuration schematically shown in FIG. 9, a heat sink 40 (41, 42) is provided on the outer face (side face in the example in this diagram) of the block part 21 of the vial rack 20. It is preferable that the heat sink is provided on the block part made of a thermally conductive metal, because the efficiency of heat exchange between the block part and the cold air in the cooling chamber is increased. Such a vial rack having the heat sink attached to the block part is configured to be capable of preferably cooling the vials containing the cell suspension using cooling gas, and is unique to the present invention and has not been available in the past. The configuration of each part of the vial rack used in the cell freezing system of the present invention can be referred to the configuration of each part of the vial rack of the present invention, such as its material and structure.

There is no particular limitation on the form of the heat sink, and the heat sink may be produced for the present invention, be a commercially available heat dissipating product (also called a heat dissipation fin), or a heat sink formed by increasing the surface area of the block part by providing protrusions and recesses in the surface of the block part. Examples of a material of the heat sink include conventionally known metal materials with high thermal conductivity, such as aluminum and aluminum alloys. When the heat sink is to be attached to the block part, an attachment method in which the heat sink comes into direct contact with the block part, such as screwing, brazing, or welding, is preferable.

### Configuration for Air Cooling Lower Face of Vial Rack

Because the lower face of the block part has a large area, heat within the block part can be readily discharged therefrom. In order to more effectively cool the vial rack with cold air in the cooling chamber, it is preferable that the cold air flowing through the cooling chamber comes into sufficient contact with the lower face of the block part and flows away. Therefore, in a preferred embodiment of the present invention, as shown in FIG. 1 as an example, support members 50 are provided to function as legs and support the block part 21 of the vial rack 20 such that the block part 21 is lifted up by the support members 50 from a placement surface, thereby exposing the lower face of the block part 21 such that the lower face can come into contact with cold air. The support member 50 may be a member separate from the vial rack, a frame of a multi-tier shelf, which will be described later, a member belonging to the cooling chamber, a member provided as a leg on the lower face of the block part of the vial rack, or a protrusion on the lower face of the block part.

FIG. 10 is a perspective view showing an example of a multi-tier shelf for arranging a large number of vial racks in a stacking direction in the cooling chamber. In the example shown in FIG. 10, a multi-tier shelf 60 has four-tier frames 61 to 64. The four-tier frames allow the four vial racks to be stacked in a state in which the four vial racks are spaced apart from each other. Each tier frame supports a surrounding region of the lower face of the block part of one vial rack. As a result, as shown in FIG. 10(a), a lower face 20b of the block part of each vial rack 20 is exposed to a central opening of the frame of each tier and is configured to come into contact with cold air.

The form of the frame is not limited to the example shown in FIG. 10(a), and a structure for supporting vial racks can be designed as appropriate such that the lower faces of the block parts of the vial racks are sufficiently exposed, such as a structure for supporting the lower faces of the block parts of the vial racks by the four corners, a structure for supporting the lower faces thereof on two sides, a structure for holding the side faces of the vial racks, a structure for supporting the vial racks using handles, or a structure in which hooks are provided on the block parts of the vial racks.

### Configuration for Favorably Transferring Heat of Interior of Block Part to Side Face

In the example shown in FIG. 10(a), the vial racks in the multi-tier shelf 60 are stacked on each other with sufficient gaps therebetween. If a gap between upper and lower vial racks is reduced in size and more vial racks are placed on a larger number of tiers, it will be difficult for cold air to pass through the gap between the upper and lower vial racks, reducing the effects of air cooling using the lower faces of the block parts of the vial racks. In order to solve this problem, the present invention proposes a configuration in which a heat conduction device such as a heat pipe or vapor chamber is used to more quickly transfer the heat in the vial racks to the side faces of the vial racks, and the heat is then removed from the side faces into the cold air. This configuration will be described below.

As shown in FIGS. 11(a) and 11(b) as exploded views, in a preferred embodiment of the present invention, heat conduction devices (vapor chambers in the diagram) 70 are embedded in the interior of the block part 21 of each vial rack such that thermal conductivity between the outer faces (in particular, the side faces) of the block part and surrounding regions of the holes is further increased. With this structure, heat entering the block part through the inner faces of the holes is transferred to the outer faces of the block part more quickly and/or in a larger amount, compared to a structure in which only a thermally conductive metal is used. As a result, as shown in FIG. 10(b), even when vial racks are densely stacked within the multi-tier shelf 60, the heat of the interiors of the vial racks can be more effectively removed by cooling the side faces of the vial racks with cold air (an arrow 12 drawn three-dimensionally for the purpose of illustration indicates the flow of cold air) passing along the sides of the multi-tier shelf 60. Also, heat around the holes can also be transferred to the lower faces of the block parts by embedding the heat conduction devices in the block parts as shown in FIG. 11.

The above heat conduction device 70 may be a heat pipe or a vapor chamber, and these may be used in combination as appropriate.

A heat pipe is a heat conduction device having a structure having a long and thin pipe with both ends closed, inside which the pressure is reduced to vacuum and in which a small amount of working fluid (such as alternatives to fluorocarbons or water) is enclosed, the heat conduction device transferring heat applied to one end of the pipe to the other end at high speed using evaporation (vaporization at one end) and condensation (liquefaction at the other end) of the working fluid, and returning on wicks of the inner wall by capillary action.

A vapor chamber is a heat conduction device having a structure in which the inside of a thin plate-shaped chamber is reduced to vacuum and a small amount of working fluid is enclosed inside, similar to the heat pipe, and heat is transferred at high speed using the same principle as the heat pipe.

Commercially available heat pipes and vapor chambers may be used, or such heat pipes and vapor chambers may be produced for the present invention. The heat conduction devices 70 can be attached to the block parts by screwing, crimping, brazing, embedding in thermal conductive grease, or through intimate contact via heat dissipation sheets.

In the example shown in FIGS. 11(a) and 11(b), in order to embed the strip-shaped vapor chambers 70 in the block part 21 of the vial rack 20, the block part 21 is constituted by two components (a main body 211 and a cover 212). The vapor chambers 70 are fitted into the grooves 71 formed in the main body 211, and the vapor chambers 70 are covered with the cover 212 to form the vial rack shown in FIG. 7. With this configuration, thermal conductivity (between the interior of the block part 21 (in particular, surrounding regions of the holes) and an outer face 211a located on one end side of the vapor chambers 70), (between the interior of the block part 21 and an outer face 211b located on the other end side of the vapor chamber 70), (between the interior and an upper face of the block part 21), and (between the interior and the lower face of the block part 21) is higher than the thermal conductivity of the original thermally conductive metal alone. The main body 211 and the cover 212 may be fixed to each other by a screw, an adhesive, welding, brazing, or the like.

### Configuration for Transferring Heat of Lower Face of Block Part to Side Face

**In** a preferred embodiment of the present invention, in an example of the configuration schematically shown in FIG. 12(a), a heat conduction device 80 is provided in a portion extending from the lower face of the block part 21 of the vial rack 20 to one side face to more efficiently conduct heat of the lower face of the block part 21 to the side face of the block part. The heat of the lower face of the block part refers to heat in a portion of the surface layer including the lower face (i.e., a lower portion of the block part).

Even when vial racks are densely stacked in the multi-tier shelf 60 as shown in FIG. 10(b), due to the heat conduction device 80 being provided, heat of the interior of the block part of each vial rack can be more efficiently removed by cooling the side faces of each vial rack, than in a structure consisting of only a thermally conductive metal.

The above heat conduction device 80 may be a heat pipe or a vapor chamber, and these may be used in combination as appropriate. Commercially available heat pipes and vapor chambers may be used, or such heat pipes and vapor chambers may be produced for the present invention.

**In** the example shown in FIG. 12(a), an L-shaped vapor chamber 80 is provided to cover the lower face and the side face of the block part 21, and transfers heat of the lower face of the block part 21 to the side face. The heat sinks 40 are attached to the vapor chamber 80 on the side face of the block part 21, and heat conducted to the side face is released into the cold air by the heat sinks 40.

**In** the example shown in FIG. 12(b), in order to embed the L-shaped vapor chamber 80 under a surface layer of the lower face of the block part 21, the block part 21 is constituted by two components (a main body 213 and a lower cover 214). The vapor chamber 80 is sandwiched between the main body 213 and the lower cover 214 to form the vial rack shown in FIG. 7. The heat sinks 40 are attached to the vapor chamber 80 on the side face of the block part 21, and heat conducted to the side face is released into the cold air by the heat sinks 40. Also, in this embodiment, the vapor chamber 80 is exposed at the bottom of the holes, and is configured to receive heat directly from the vials. The main body 213 and the lower cover 214 may be fixed to each other by a screw, an adhesive, welding, brazing, or the like.

### Temperature Sensor

**In** a preferred embodiment of the present invention, a temperature sensor for measuring the temperature of the block part of the vial rack is attached to the block part. The temperature sensor is configured to be capable of communicating temperature-related data with a control unit of the gas-cooling type freezer or an external device in a case where the vial rack is placed in the cooling chamber of the gas-cooling type freezer. This enables precise temperature control according to the temperature of the vial rack, and allows the temperature of the vial rack to be recorded over time. The external device may be any device capable of communicating temperature-related data with the temperature sensor via wired or wireless communication, and may be any one of various devices such as a data recording device (such as a temperature logger), an external computer, a communication device and a data recording device connected thereto, a server computer on the Internet, and the like.

### Configuration for Individually Controlling Temperatures of Individual Vial Racks

When a large number of vial racks accommodating vials are placed on the multi-tier shelf and a large number of multi-tier shelves are arranged in a large cooling chamber as shown in FIG. 10(b), the cooling conditions of the vial racks may differ and the temperature drop rates may also differ depending on their positions in the cooling chamber. **In** order to solve this problem, the present invention proposes a unique configuration in which the vial racks can be individually and independently heated or cooled. This configuration will be described below.

As shown in FIG. 13 as an example, in a preferred embodiment of the present invention, one or preferably both of the heater 90 and the Peltier element 91 is provided on the block part 21 (in particular, on its lower face 20b) of each vial rack 20 or at an installation position for each vial rack in the cooling chamber (a frame 65 of each tier of the multi-tier shelf in the diagram). One or both of the heater 90 and the Peltier element 91 are connected to, for example, a control unit of the gas-cooling type freezer, and are configured such that the temperatures of the vial racks are separately adjustable. As a result, even if there are variations in cooling conditions among a large number of vial racks placed in the cooling chamber, individual vial racks can be independently heated or cooled, making it possible to finely adjust the temperatures of individual vial racks while cooling the entire cooling chamber using a fan or the like.

The above configuration, in which the temperatures of the vial racks can be separately adjusted using the heater or the Peltier element, can be realized by combining the vial racks each having the block part made of a thermally conductive metal as a main body, with the gas-cooling type freezer.

The heater and the Peltier element may be attached to the lower face of the block part of the vial rack, or may be attached to the frame 65 of each tier of the multi-tier shelf.

The heater and/or the Peltier element attached to the frame 65 of each tier of the multi-tier shelf comes into contact with the block part (preferably the lower face) of the vial rack when the vial rack is placed on the frame 65. As shown in FIG. 13, preferably, the heater and/or the Peltier element covers only a portion of one face of the block part, leaving a region that can come into contact with cold air. Electrical wiring for supplying power to the heater and/or the Peltier element may be configured to achieve appropriate connection when vial racks and multi-tier shelves are placed in the cooling chamber. The heaters and/or the Peltier elements may be individually heated or cooled according to a predetermined program, or under the control of temperature where the output of the heaters and/or the Peltier elements is adjusted based on the temperatures of the vial racks measured using the above temperature sensors. The program is a data set of cooling conditions (e.g., temperature and a time period in which the temperature is maintained) to obtain a predetermined temperature change profile. The program may be constructed by a control circuit, or if the control device is a computer, may be constructed in the form of computer software.

The vial rack used in the cell freezing system or the vial rack of the present invention not only favorably absorbs the latent heat generated when the cell suspension in the vials is frozen, but is also useful for suppressing temperature variations in the vials when a large number of vials are to be transferred. For example, conventionally, when a cell suspension or the like is placed in vials and cryopreserved, a large number of vials are inserted into a resin vial rack for cryopreservation, known as a freezer box or the like, and the freezer box is then placed in an ultra-low temperature storage container containing liquid nitrogen. When the freezer box is removed from such an ultra-low temperature storage container into the chamber, the temperature of the freezer box will rise rapidly. At this time, the degree of temperature rise of the vials varies depending on the positions in the freezer box, and the temperature of peripheral vials adjacent to an outer peripheral wall (especially, vials at the four corners) is higher than the temperature of a vial near the center. **In** contrast, when the vial rack used in the present invention or the vial rack of the present invention is used as a freezer box, the temperature of each part in the vial rack is made uniform due to rapid heat transfer, and temperature variation of the products in a large number of vials accommodated in the vial rack is also suppressed. Also, if the vial rack used in the present invention or the vial rack of the present invention is used as a vial transport device, the vials can be cooled through the vial rack, for example, by immersing the lower part of the vial rack in shallow liquid nitrogen contained in a tray, thus easily maintaining the inserted vials at low temperature. This makes it possible to easily maintain vials at low temperature even in the operation of repacking the vial rack accommodating the cooled vials from a storage box to a shipping box (operation outside the cooling chamber), for example.

### Gas-Cooling Type Freezer

The "gas-cooling type freezer" used in the present invention is a freezer that is provided with a cooling chamber and is configured to cool a freezing target placed in the cooling chamber using a low-temperature cooling gas. The gas-cooling type freezer may be used not only as a device for freezing a freezing target, but also as a refrigerator that refrigerates the freezing target at a temperature (e.g., 0°C to 10°C or the like) higher than a freezing temperature by adjusting the temperature of the cooling gas. The gas-cooling type freezer may be designed and produced for the present invention, or may be a commercially available product.

When the cell suspension in the vials inserted in a vial rack is to be frozen using the gas-cooling type freezer, low-temperature cooling gas capable of freezing the cell suspension is sent into the cooling chamber. The freezing temperature of the cell suspension varies depending on the components of the cell suspension and is usually -60°C to -80°C, and it is preferable that the temperature of the cooling gas sent into the cooling chamber is sufficiently lower than the freezing temperature. There are no particular limitation on the cooling gas sent into the cooling chamber and its temperature, and examples thereof include low-temperature air (about -194°C) immediately after vaporization from liquefied air, low-temperature nitrogen gas (about -196°C) immediately after vaporization from liquid nitrogen, low-temperature carbon dioxide (about -79°C) immediately after vaporization from dry ice or liquefied carbon dioxide, and refrigerants (about -82°C) such as fluorocarbons (or alternatives to fluorocarbons). In particular, nitrogen gas obtained from liquid nitrogen is a preferred cooling gas because it has an ultra-low temperature and is easy to handle.

The vaporized low-temperature nitrogen gas or carbon dioxide gas may be injected directly into the cooling chamber, or may be mixed with air and injected into the cooling chamber. When air is used as the cooling gas, a separate cooling device is provided for cooling the air to a sufficiently low temperature and sending the air into the cooling chamber.

The gas-cooling type freezer shown in FIG. 20 as an example is configured such that, as indicated by the arrows, cold air is circulated within the cooling chamber. In the case of more powerful cooling, nitrogen gas at -196°C is injected into the cooling chamber, and the gas in the cooling chamber is exhausted to the outside through an exhaust port in an amount equal to the amount of nitrogen gas that has flowed in. The temperature inside the cooling chamber is preferably controlled by precisely adjusting the ON/OFF of the nitrogen gas injection and by stirring the cold air in the cooling chamber. Examples of such gas-cooling type freezer include CryoMed available from ThermoFisher, Kryo available from Planar, controlled-rate freezer (CRF) available from Biolife Solutions, and CRF available from Taiyo Nippon Sanso Corporation. All of these freezers are programmable freezers, which will be described later.

### Capacity of Cooling Chamber

The capacity of the cooling chamber of the gas-cooling type freezer is not particularly limited, and can be selected as appropriate depending on the size of vial racks to be arranged for mass production, the number of vial racks, and the like, from the size thereof that can be placed on a desk to a size of a warehouse that a person can enter.

### Fan

The number of fans, flow rate, flow direction, and other configurations for injecting cold air into the cooling chamber of the gas-cooling type freezer can be any of those known in the art. A large number of fans may be provided facing in various directions such that cold air is evenly distributed to various positions in the cooling chamber.

### Programmable Freezer

The gas-cooling type freezer is preferably a programmable freezer capable of varying cooling capacity according to a preset program. The program is a data set of cooling conditions (e.g., one or more sets of cooling chamber temperatures and time periods in which the temperature is maintained) for changing the temperature of the cooling chamber according to a predetermined temperature change profile. The programmable freezer is configured to change the temperature of the cooling chamber instantly (or at a predetermined gradient) according to a data set defined in the program and to maintain a predetermined temperature for a predetermined period of time. The program may be constructed by a control circuit in which the temperature sensor, a timer, a drive device that changes the output of a cooling source, and the like are combined, or if the control device is a computer, the program may be constructed by computer software. By using the programmable freezer, the temperature rise of the cell suspension caused by latent heat can be more effectively suppressed by changing the preset cooling capacity, making effects of the present invention more significant.

Many commercially available cell preservation solutions solidify at about -5°C to about -10°C, including in a supercooled state, and therefore rapid cooling is often performed in this temperature range. A typical programmable freezer is configured such that a target temperature in the cooling chamber can be set. Thus, the operation of cooling vials containing a cell suspension in the programmable freezer is performed, for example, according to the following steps mentioned as examples.

(Step 1) Pre-cooling of vials in the cooling chamber and stabilization of the temperature: The temperature in the cooling chamber is set to -10°C, and a period in which the temperature is maintained at -10°C is set to 20 minutes. Cold air is constantly circulated. In this environment, each vial is cooled from room temperature to the freezing temperature of -10°C, and moves to the next step just before the release of the latent heat is started. The generation of latent heat in Step 2 can be more effectively suppressed by adjusting as appropriate a time period in which the temperature is maintained at -10°C in Step 1 in accordance with the number of vials to be frozen, the amount of liquid, and the like.

(Step 2) Dealing with latent heat: The temperature in the cooling chamber is set to -60°C, and the release of the latent heat is started. This powerful cooling suppresses the temperature rise caused by latent heat, and the cell suspension in all of the vials freezes. A period in which the temperature is maintained at -60°C is set to 15 minutes.

(Step 3) Slow cooling: The temperature in the cooling chamber is set to -50°C. The interior of the cooling chamber is then cooled to a set temperature (about -80°C) such that the temperature of the vials drops at a constant rate (approximately -1 to -3 (°C/min)).

(Step 4) Ordinary cooling (optional): The temperature in the cooling chamber is maintained at -80°C.

Next, a cell freezing method according to the present invention will be described.

The cell freezing method is a method of freezing a cell suspension using the above-mentioned cell freezing system of the present invention. As shown in FIG. 1, the cell freezing method includes a step of preparing a vial rack 20 into which the vials 30 containing the cell suspension 31, which is a freezing target, are inserted, and a cooling step of cooling the vial rack 20 into which the vials 30 are inserted, in the cooling chamber 11 of the gas-cooling type freezer 10, and thereby cooling and freezing the cell suspension 31, which is the freezing target.

In the step of preparing the vial rack into which the vials are inserted, the cell suspension may be first placed in the vials and then the vials may be inserted into the vial rack, or empty vials may be first inserted into the vial rack and then the cell suspension may be poured into the vials.

In the cooling step, the gas-cooling type freezer 10 cools the block part 21 of the vial rack 20 using cold air at a temperature at which the cell suspension 31 freezes. The cooled block part 21 effectively removes heat from the vials 30 and the cell suspension 31 therein, thus cooling and freezing the cell suspension 31.

### Examples

### Example 1

In this example, the cell freezing system of the present invention was actually constructed using a programmable freezer and a vial rack made of an aluminum alloy, and preferred cooling conditions for the programmable freezer were determined. Then, it was confirmed that the cell freezing system of the present invention favorably cooled a large number of vials at once even when the gas-cooling type freezer was used. Details of the configuration of the cell freezing system, the vials, the cell preservation solution, and the like are as follows.

### (Gas-Cooling Type Freezer)

A programmable freezer (CryoMed Controlled-Rate Freezer, 7474) manufactured by Thermo Scientific.

The programmable freezer is configured such that the temperature change inside the cooling chamber over time can be preset as a program, and is also configured such that the amount and duration of cooling gas (nitrogen gas at about -196°C) injected into the cooling chamber is adjusted to change the temperature inside the cooling chamber over time in accordance with the program.

### (Vial Rack)

The thermally conductive metal, which is the material of the vial rack, is aluminum alloy A5052 specified in JIS H4000: 2014.

In this example, the vial rack having the form shown in FIG. 7 was used. The basic shape of the block part excluding the handle was a rectangular cuboid (a long side of the upper face was 122 mm, a short side of the upper face was 108.5 mm, and the height was 20 mm), and was formed by cutting a block made of an aluminum material.

On the upper face, 16 holes (round columnar straight holes) for inserting vials were arranged in a square matrix of 4 rows and 4 columns. The inner diameter of each hole was 22.3 mm, the depth of the hole was 18 mm, and center-to-center dimensions between adjacent holes were 30 mm in a long side direction and 25.5 mm in a short side direction. In this example, no spacers were provided in the holes.

### (Vial)

A container part of the vial conforms to ISO 8362-1 6R, and is made of a cyclic olefin polymer.

The basic outer shape of the body part of the vial is round columnar, and its outer diameter is 22 mm.

The height from the lower face to the shoulder of the body part is 26 mm.

The vial lid is a commercially available product (RayDyLyo CTO 20Φ) constituted by a rubber stopper and a plastic cap covering the stopper, and has a built-in rubber stopper conforming to ISO 8362-2.

### (Cell Preservation Solution)

The cell preservation solution is an aqueous solution containing dimethyl sulfoxide as a main component, and its freezing point is about -5°C. Into each vial, 5 mL of the cell preservation solution was poured, and each vial was sealed with a lid.

### (Configuration for Arranging Vial Racks in Cooling Chamber)

Vials containing the cell preservation solution were respectively inserted into 16 holes provided in one vial rack. Sixteen sets of vial racks with vials inserted in this manner (the total number of vials was 256) were prepared and placed in the cooling chamber of the programmable freezer. When the vial racks were placed, four shelves (60a, 60b, 60c, 60d) were used as shown in FIG. 14(a). The structure of each shelf was a frame structure as shown in FIG. 10(a), and shelves with four tiers were used in this example. As shown in FIG. 14(a), the four shelves were arranged in a 2×2 square matrix pattern and 16 sets of vial racks were arranged with appropriate spacing between vertically adjacent vial racks and between horizontally adjacent vial racks.

### (Shelf Board)

As schematically shown in FIG. 15, a shelf board 60p of each tier on which each vial rack is placed has opening windows (through holes) 60h at corresponding positions directly below the vials. This exposes the lower face of each vial rack (in particular, corresponding regions directly below the vials) to cold air.

### (Attachment of Thermocouples to Vials and Temperature Measurement System)

In order to measure the temperatures on the outer circumferential side and the central side of each vial rack and to measure the temperature of each part inside the cooling chamber, through holes were provided in the lids of three vials selected as appropriate from the 16 vials inserted in each vial rack, and a temperature measuring part of a thermocouple (T thermocouple, 9811, manufactured by Hioki E.E. Corporation) was inserted. FIG. 16 shows an arrangement pattern of the three vials provided with thermocouples, out of the 16 vials in each vial rack placed on the four shelves (60a, 60b, 60c, 60d). Black circles represent the vials provided with the thermocouples, and white circles represent the vials provided with no thermocouples. The temperature of the cell preservation solution in the vial was measured using the thermocouple over time and recorded using an external temperature logger (Memory HiLogger, LR8450, manufactured by Hioki E.E. Corporation). The temperature logger used was of a multi-channel type in order to evaluate an effect of a temperature distribution inside a programmable freezer cabinet.

### (Overview of Program Optimization)

A non-optimized tentative program was set, the temperature logger was started to record temperature data, and the programmable freezer was started at the same time to cool the interior of the cooling chamber at room temperature according to the tentative program, thereby freezing the cell preservation solution in each vial. Then, the operation of modifying details of the program based on the temperature changes was repeated to obtain an optimized program. The details of this optimization are as follows.

### (Tentative Program Setting Values)

Step 1 (Cooling from room temperature to freezing temperature):
Set temperature inside the cooling chamber: -10°C (the temperature inside the cooling chamber drops rapidly from room temperature to a set temperature in about 1 minute, and the temperature inside the vial drops under the influence of the temperature inside the cooling chamber)
A time period in which the temperature is maintained at -10°C: 20 minutes

Step 2 (Rapid cooling to induce solidification and suppress temperature rise caused by latent heat):
Set temperature inside the cooling chamber: -60°C
A time period in which the temperature is maintained at -60°C: 15 minutes

Step 3 (Recovery from rapid cooling):
Set temperature inside the cooling chamber: -50°C
A time period in which the temperature reaches -50°C: 1 minute

Step 4 (Slow cooling to suppress freezing-induced cell damage):
The temperature inside the cooling chamber is cooled from -50°C to -80°C over 15 minutes at a temperature drop rate of -2°C/min.

Step 5 (Ordinary cooling):
Set temperature inside the cooling chamber: -80°C
A time period in which the temperature is maintained at -80°C: 30 minutes

### (Modification of Program)

After the cooling according to the above tentative program was completed, the records of the temperature logger were analyzed to check a temperature change in the cell preservation solution in each vial. From the temperature change in the cell preservation solution, the time when latent heat was generated (time elapsed from the start of cooling) and the amount of temperature rise at that time were obtained, and the time of Step 1 above was corrected such that the rapid cooling of Step 2 above would be started just before the time when latent heat was generated (the time when the temperature inside the vial reached 0°C).

### (Further Modification of Program)

The vial was cooled in the same manner as above, except for using the above modified program, and the target temperature of the rapid cooling in Step 2 and time taken therefor were optimized to suppress latent heat while preventing excessive cooling. Further, the cooling conditions from Step 3 onwards were set such that the vial would be cooled slowly after the release of latent heat had ended. The slow cooling rate was set such that the temperature inside the vial was about -1°C/min to about -3°C/min. It is desirable to actually freeze the cells and evaluate separately an optimal slow cooling rate and an optimal acceptable range after the generation of latent heat.

The shelf arrangement was modified based on variation in the temperature of each vial. By arranging the shelves at positions excluding positions where vials were cooled relatively quickly or slowly, temperature variations among a large number of vials were further suppressed.

As described above, it was found that with the cell freezing system of the present invention, the freezing targets in a large number of vials were favorably frozen at once using the gas-cooling type freezer.

### Example 2

In this example, the configuration of the cell freezing system used in Example 1 was reused, and a comparison was made between the vial rack having the block part made of a thermally conductive metal (the vial rack of Example 1) and a conventional frame-type vial rack (a vial rack of Comparative Example).

### (Conventional Frame-Type Vial Rack)

As a conventional vial rack, a vial rack was used, which had a frame structure in which grid plates of three tiers were fixed at intervals by supports (spacers) at the four corners, as shown in FIG. 19(a) using reference numeral 120. In the vial rack, cold air enters the vial rack from the sides thereof, and the vials are directly cooled by the cold air. The arrangement pattern of openings of the through holes provided in the grid plates of the uppermost and second tiers was a 4×4 square matrix, which was the same as the arrangement pattern of the openings of the vial rack of Example 1. The grid plate of the lowermost tier also had through holes, but the inner diameter of the opening of each through hole was smaller than the outer diameter of the body part of a vial such that the vial did not fall out. Also, a configuration was realized in which the temperature measuring parts of the thermocouples were inserted into vials inserted into the conventional vial rack in the same arrangement pattern as in Example 1, and thus the temperature of the cell preservation solution contained in the vials could be recorded over time using an external temperature logger.

### (Vial Rack Arrangement for Comparison)

Out of the total of 16 sets of vial racks placed on the four shelves (60a, 60b, 60c, 60d) in the cell freezing system used in Example 1, 8 sets of vial racks were replaced with the conventional vial rack. In this replacement, as shown in FIG. 14(b), a vial rack 20A of Example 1 and a vial rack 20B of Comparative Example were arranged adjacent to each other in the up-down direction and the lateral direction. The vials accommodated in the vial racks are all the same as those in Example 1. In FIG. 14(b), for the purpose of illustration, only the vial racks 20B of Comparative Example are hatched. The arrangement pattern of the four shelves is a 2×2 square matrix pattern, similar to Example 1.

### (Temperature Change Inside Vials through Rapid Cooling)

Four shelves were arranged in the cooling chamber of the programmable freezer in the arrangement pattern shown in FIG. 14(b), and rapid cooling was performed. The program for the rapid cooling was as follows. Because the purpose of this example is to compare temperature changes caused by the generation of latent heat, the temperature control (temperature rise, slow cooling) after the generation of latent heat is omitted.

### Step 1

Set temperature inside the cooling chamber: -20°C
A time period in which the temperature is maintained at -20°C: 5 minutes

### Step 2

Set temperature inside the cooling chamber: -10°C
A time period in which the temperature is maintained at -10°C: 10 minutes

### Step 3

Set temperature inside the cooling chamber: -80°C
A time period in which the temperature is maintained at -80°C: 20 minutes

After the cooling according to the above program was completed, the records of the temperature logger were analyzed to check a temperature change in the cell preservation solution in each vial.

FIG. 17 is a graph diagram showing typical temperature changes of the cell preservation solutions in the vial racks of Example 1 and Comparative Example. Each graph diagram also shows the temperature change inside the cooling chamber. FIG. 17(a) is a graph diagram showing the temperature changes in the cell preservation solution inside the vials in the vial racks of Example 1, and FIG. 17(b) is a graph diagram showing the temperature changes in the cell preservation solution inside the vials in the conventional vial racks.

As is clear from a comparison of the graph diagrams shown in FIGS. 17(a) and 17(b), regarding the temperature changes inside the vials inserted into the vial racks of Example 1, compared to the temperature changes inside the vials inserted into the vial racks of Comparative Example, a steep temperature rise caused by latent heat was suppressed, resulting in a gradual temperature drop that was closer to a temperature drop at a constant rate. Furthermore, these results revealed that by using the cell freezing system of the present invention, even if the cooling chamber is simply cooled rapidly, the larger heat capacity of the block parts of the vial racks can suppress a rapid temperature rise caused by latent heat.

### Example 3 Cryopreservation of Cells

In this example, the configuration of the cell freezing system used in Example 1 was utilized to cryopreserve the cells.

Cultured cells (iPS cell-derived NK cells) were collected using an appropriate method. The resulting cell suspension was then washed and concentrated using a cell concentration and washing system (Kaneka Corporation).

The cell preservation solution used in Example 1 was then added to prepare a cell suspension. The cell density may be any desired value, and was usually set to 1×10⁵ to 1×10⁸ cells/mL, and more preferably 5×10⁵ to 5×10⁷ cells/mL.

The cell suspension prepared above was aseptically dispensed into vials similar to those in Example 1 and sealed.

Then, 256 of the vials containing the cell suspension were prepared, and these vials were inserted into 16 vial racks in the same manner as in Example 1, placed on the four shelves (each shelf has four tiers) as shown in FIG. 14(a), and placed in the programmable freezer. Then, the programmable freezer was operated to freeze the cell suspension inside the vials according to a cooling program optimized based on Example 1.

After the program was completed, the vials were removed from the programmable freezer, and placed in a vial box, and stored under liquid nitrogen vapor.

The cells cryopreserved using the configuration of the cell freezing system of the present invention as described above had a suppressed temperature rise caused by latent heat during the freezing stage, which strongly suggests that, for example, the survival rate (viability) of cells when activated is improved compared to cells cryopreserved using a conventional method.

### Industrial Applicability

According to the present invention, a large amount of high-quality frozen cells can be **liquid** surface at once in a process for producing cellular pharmaceuticals. Therefore, the present invention plays an extremely important role in the production of cellular pharmaceuticals.

### Description of Reference Numerals

- 10: Gas-cooling type freezer
- 11: Cooling chamber
- 12: Cooling gas (cold air)
- 20: Vial rack
- 21: Block part made of thermally conductive metal
- 30: Vial
- 31: Cell suspension
- C1: Heat from vial rack to cold air

The present application claims the benefit of priority based on Japanese Patent Application No. 2022-131339 filed in Japan (filing date: August 19, 2022) in Japan, the disclosure of which is incorporated herein in its entirety by reference.

## Claims

1. A cell freezing system for freezing a cell suspension by cooling one or more vials containing the cell suspension and inserted into a vial rack,
the cell freezing system comprising:
a gas-cooling type freezer having a cooling chamber; and
the vial rack to be placed in the cooling chamber,
wherein the vial rack has a block part made of a thermally conductive metal as a main body, the block part having one or more holes for inserting a body part of the vial at least up to a liquid surface of the cell suspension, and
during use, the gas-cooling type freezer cools the vial rack placed in the cooling chamber in a state in which the vials are inserted into the vial rack, using cooling gas, and the cooled vial rack cools the vial and the cell suspension in the vial through an inner face of the hole in the block part.

2. The cell freezing system according to claim 1,
wherein the thermally conductive metal has a thermal conductivity of 100 (W/(m·K)) or more.

3. The cell freezing system according to claim 1 or 2,
wherein the thermally conductive metal is aluminum, an aluminum alloy, copper, a copper alloy, magnesium, or a magnesium alloy.

4. The cell freezing system according to any one of claims 1 to 3,
wherein a basic shape of the block part of the vial rack is a three-dimensional shape having a flat upper face,
a plurality of the holes extend from the upper face into the block part, and
openings of the plurality of holes are positioned on the upper face in a predetermined arrangement pattern.

5. The cell freezing system according to claim 4,
wherein the three-dimensional shape is a rectangular cuboid or a cube.

6. The cell freezing system according to any one of claims 1 to 5,
wherein a basic outer shape of the body part of the vial is round columnar, and a basic shape of an internal space of the hole for inserting the body part is round columnar, and
a difference between an inner diameter of the hole and an outer diameter of the body part of the vial is 0.1 to 3 mm.

7. The cell freezing system according to any one of claims 1 to 6,
wherein the inner face of the hole is surface treated to have higher emissivity.

8. The cell freezing system according to any one of claims 1 to 7,
wherein the hole is a blind hole having a bottom face therein, and
the inner face of the hole has one or more grooves extending from the opening of the hole to the bottom face, and when the body part of the vial is inserted into the hole, air inside the hole is allowed to escape to an outside through the grooves.

9. The cell freezing system according to claim 8,
wherein the groove provided on an inner circumferential surface of the hole and the groove provided in an adjacent hole are integrally connected to each other to form a single slit, thereby allowing adjacent holes to be in communication with each other.

10. The cell freezing system according to any one of claims 1 to 9,
wherein a heat sink is provided on an outer face of the block part to further increase efficiency of heat exchange between the block part and the cooling gas inside the cooling chamber.

11. The cell freezing system according to any one of claims 1 to 10,
wherein an interior of the block part is provided with one or both of a heat pipe and a vapor chamber to further increase thermal conductivity between an outer face of the block part and a periphery of the hole.

12. The cell freezing system according to any one of claims 1 to 11,
wherein the block part is provided with one or both of a heat pipe and a vapor chamber such that heat of a lower face of the block part is more efficiently conducted to a side face of the block part.

13. The cell freezing system according to any one of claims 1 to 12,
wherein a temperature sensor for measuring a temperature of the block part is attached to the block part of the vial rack, and the temperature sensor is configured to be capable of communicating temperature-related data with a control unit of the gas-cooling type freezer or an external device in a case where the vial rack is placed in the cooling chamber of the gas-cooling type freezer.

14. The cell freezing system according to any one of claims 1 to 13,
wherein the cooling chamber has an interior space sufficiently large to allow a plurality of vial racks to be placed, and
one or both of a heater and a Peltier element are provided on the block part of each vial rack or at an installation position for each vial rack in the cooling chamber, to allow temperatures of the vial racks to be individually adjustable.

15. The cell freezing system according to any one of claims 1 to 14,
wherein the gas-cooling type freezer is a programmable freezer capable of varying cooling capacity according to a preset program, and
the programmable freezer is configured to increase the cooling capacity during a period in which latent heat is released, to suppress a temperature rise of the cell suspension caused by release of the latent heat when the cell suspension is frozen.

16. A vial rack for use in the cell freezing system according to any one of claims 1 to 15, the vial rack comprising
a block part made of a thermally conductive metal as a main body, wherein the block part has one or more holes for inserting the body part of the vial containing the cell suspension at least up to the liquid surface of the cell suspension.

17. The vial rack according to claim 16,
wherein a heat sink is provided on an outer face of the block part to further increase efficiency of heat exchange between the block part and the cooling gas.

18. The vial rack according to claim 16 or 17,
wherein an interior of the block part is provided with one or both of a heat pipe and a vapor chamber to further increase thermal conductivity between an outer face of the block part and a periphery of the hole.

19. The vial rack according to any one of claims 16 to 18,
wherein the block part is provided with one or both of a heat pipe and a vapor chamber to more efficiently conduct heat of a lower face of the block part to a side face of the block part.

20. A cell freezing method using the cell freezing system according to any one of claims 1 to 15, the method comprising:
a step of preparing a vial rack for the cell freezing system, a vial containing a cell suspension to be frozen being inserted in the vial rack; and
a cooling step of cooling the vial rack into which the vial is inserted, in the cooling chamber of the gas-cooling type freezer of the cell freezing system, to cool and freeze the cell suspension,
wherein, in the cooling step, the gas-cooling type freezer cools the block part of the vial rack to a temperature at which the cell suspension freezes, using cooling gas, and the cooled block part cools the vial and the cell suspension in the vial through the inner face of the hole, thereby freezing the cell suspension.
